# EUROPEAN PATENT APPLICATION

(11) **EP 3 711 667 A1**
(43) Date of publication of application: **23.09.2020**
(21) Application number: 18837274.2
(22) Date of filing: 30.07.2018
(51) Int. Cl.: A61B 5/11, A61B 5/0488

(54) **SYSTEM, METHOD, AND PROGRAM FOR RECOGNIZING MYOELECTRIC SIGNAL-ORIGINATING MOTION**

(30) Priority: 28.07.2017 JP 2017147242
(71) Applicant: Meltin MMI Co., Ltd., Tokyo 1040033 (JP)
(72) Inventor: SEKI Tatsuya, Chuo-ku, Tokyo 1040033 (JP); ISHII Toshiaki, Chuo-ku, Tokyo 1040033 (JP); YAMADA Hiroyuki, Koganei-shi Tokyo 184-0011 (JP); TAMURA Fumiyo, Koganei-shi Tokyo 184-0011 (JP); KIKUTANI Takeshi, Koganei-shi Tokyo 184-0011 (JP)
(74) Representative: Uexküll & Stolberg
(86) International application number: PCT/JP2018/028492
(87) International publication number: WO 2019/022259

(57) **Abstract**

A system (**10**) for evaluating a movement of a subject comprises: detection means (**111**, **112**) for detecting a myoelectric signal indicating an activity of a muscle when a subject performs a movement intended to be a predetermined movement comprising a jaw movement, a tongue movement, or a throat movement; processing means (**221**) for outputting a processed myoelectric signal by applying predetermined processing on the detected myoelectric signal; and determination means (**222**) for determining a motion level in the predetermined movement of the subject, based on a pattern contained in the processed myoelectric signal.

## Description

### [Technical Field]

The present invention relates to a system, a method, and a program for recognizing a myoelectric signal-originating motion.

### [Background Art]

An oral sensor capable of detecting a motion of tongue movement has been developed (see, for example, Patent Literature 1).

### [Citation List]

### [Patent Literature]

[PTL 1] International Publication No. WO 2013/085038

### [Summary of Invention]

### [Technical Problem]

Since oral sensors are installed inside the oral cavity to measure the pressure applied by the tongue in the oral cavity, motions of tongue movement that do not involve a contact between the tongue and the sensor, motions of jaw movement or throat movement that are not tongue movement could not be detected.

The objective of the present invention is to provide a system, a method, and a program for recognizing myoelectric signal-originating motions comprising at least two of a motion of a jaw movement, a motion of a tongue movement, and a motion of a throat movement.

Another objective of the present invention is to provide a system, a method, and a program for evaluating a movement of a subject.

### [Solution to Problem]

The system for recognizing a myoelectric signal-originating motion of the invention comprises detection means for detecting a myoelectric signal indicating activities of a plurality of muscles, processing means for outputting a processed myoelectric signal by applying predetermined processing on the detected myoelectric signal, and determination means for determining which one or more of a plurality of patterns of motions comprising at least two of a pattern of a motion of a jaw movement, a pattern of a motion of a tongue movement, and a pattern of a motion of a throat movement is a pattern contained in the processed myoelectric signal.

In one embodiment, the predetermined processing comprises frequency analysis processing, and the processed myoelectric signal comprises time series data for a frequency distribution of the myoelectric signal.

In one embodiment, the frequency analysis processing comprises a short-time FFT.

In one embodiment, the predetermined processing further comprises signal intensity extraction processing, and the processed myoelectric signal further comprises time series data for signal intensities of the myoelectric signal.

In one embodiment, the determination means comprises learning means for learning at least two of a supervisory pattern of a motion of a jaw movement, a supervisory pattern of a motion of a tongue movement, and a supervisory pattern of a motion of a tongue movement, calculation means for calculating a degree of similarity between a pattern contained in the processed myoelectric signal and the supervisory pattern, and judgment means for judging whether the degree of similarity exceeds a predetermined threshold value.

In one embodiment, the detection means comprises a myoelectric sensor comprising two pairs of measurement electrodes, and the two pairs of measurement electrodes are configured to be able to detect a myoelectric signal indicating activities of at least two of a jaw muscle, a tongue muscle, and a throat muscle.

The method for recognizing a myoelectric signal-originating motion of the invention comprises: detecting a myoelectric signal indicating activities of a plurality of muscles; outputting a processed myoelectric signal by applying predetermined processing on the detected myoelectric signal; and determining which one or more of a plurality of patterns of motions comprising at least two of a pattern of a motion of a jaw movement, a pattern of a motion of a tongue movement, and a pattern of a motion of a throat movement is a pattern contained in the processed myoelectric signal.

The program for recognizing a myoelectric signal-originating motion of the invention is executed in a computer apparatus comprising a processor unit, wherein the program, when executed, makes the processor unit perform processing comprising: receiving a myoelectric signal indicating activities of a plurality of muscles; outputting a processed myoelectric signal by applying predetermined processing on the received myoelectric signal; and determining which one or more of a plurality of patterns of motions comprising at least two of a pattern of a motion of a jaw movement, a pattern of a motion of a tongue movement, and a pattern of a motion of a throat movement is a pattern contained in the processed myoelectric signal.

The present invention provides, for example, the following.
(Item 1)
   A system for evaluating a movement of a subject, the system comprising:
   detection means for detecting a myoelectric signal indicating an activity of a muscle when a subject performs a movement intended to be a predetermined movement comprising a jaw movement, a tongue movement, or a throat movement;
   processing means for outputting a processed myoelectric signal by applying predetermined processing on the detected myoelectric signal; and
   determination means for determining a motion level in the predetermined movement of the subject, based on a pattern contained in the processed myoelectric signal.
(Item 2)
   The system of item 1, wherein the predetermined processing comprises frequency analysis processing, and the processed myoelectric signal comprises time series data for a frequency distribution of the myoelectric signal.
(Item 3)
   The system of item 2, wherein the frequency analysis processing comprises a short-time FFT.
(Item 4)
   The system of item 2 or 3, wherein the predetermined processing further comprises signal intensity extraction processing, and the processed myoelectric signal further comprises time series data for signal intensities of the myoelectric signal.
(Item 5)
   The system of any one of items 1 to 4, wherein the determination means is subjected to learning processing by using a supervisory pattern of a motion corresponding to the predetermined movement among a supervisory pattern of a motion of a jaw movement, a supervisory pattern of a motion of a tongue movement, and a supervisory pattern of a motion of a tongue movement, so that a degree of similarity to a myoelectric signal generated when there is no impairment in the predetermined movement is outputted,
   wherein the determination means comprises:
   calculation means for calculating a degree of similarity between the pattern contained in the processed myoelectric signal and the supervisory pattern.
(Item 6)
   The system of item 5, wherein the determination means further evaluates the presence or absence of an impairment in the predetermined movement based on the degree of similarity.
(Item 7)
   The system of item 5 or 6, wherein the learning processing is applied using a supervisory pattern of a motion of a jaw movement, a supervisory pattern of a motion of a tongue movement, and a supervisory pattern of a motion of a throat movement.
(Item 8)
   The system of item 7, wherein the learning processing is applied using at least a supervisory pattern of a mouth opening and closing motion associated with the motion of a jaw movement, at least a supervisory pattern of a tongue raising motion, a supervisory pattern of a right side translation motion of the tongue, a supervisory pattern of a left side translation motion of the tongue, and a supervisory pattern of a forward extension motion of the tongue associated with the motion of a tongue movement, and at least a supervisory pattern of a dry swallowing motion associated with the motion of a throat movement.
(Item 9)
   The system of item 8, wherein the learning processing is applied by further using a supervisory pattern of a tapping motion associated with the motion of a jaw movement and a supervisory pattern of a left and right translation motion of the tongue associated with the motion of a tongue movement.
(Item 10)
   The system of any one of items 1 to 9,
   wherein the detection means comprises a myoelectric sensor comprising two pairs of measurement electrodes, and
   wherein the two pairs of measurement electrodes are configured to be able to detect a myoelectric signal indicating an activity of a jaw muscle, a tongue muscle, or a throat muscle.
(Item 11)
   A method for evaluating a movement of a subject, the method comprising:
   detecting a myoelectric signal indicating an activity of a muscle when a subject performs a movement intended to be a predetermined movement comprising a jaw movement, a tongue movement, or a throat movement;
   outputting a processed myoelectric signal by applying predetermined processing on the detected myoelectric signal; and
   determining a motion level in the predetermined movement of the subject, based on a pattern contained in the processed myoelectric signal.
(Item 12)
   A program for evaluating a movement of a subject, the program executed in a computer apparatus comprising a processor unit, wherein the program, when executed, causes the processor unit to perform processing comprising:
   receiving a myoelectric signal indicating an activity of a muscle when a subject performs a movement intended to be a predetermined movement comprising a jaw movement, a tongue movement, or a throat movement;
   outputting a processed myoelectric signal by applying predetermined processing on the received myoelectric signal; and
   determining a motion level in the predetermined movement of the subject, based on a pattern contained in the processed myoelectric signal.
(Item 13)
   A classifier used for evaluating a movement of a subject, the classifier comprising:
   detection means for detecting a myoelectric signal indicating an activity of a muscle when a subject with no impairment in a predetermined movement comprising a jaw movement, a tongue movement, or a throat movement performs the predetermined movement;
   processing means for outputting a processed myoelectric signal by applying predetermined processing on the detected myoelectric signal; and
   learning means for learning a pattern contained in the processed myoelectric signal as a pattern in the predetermined movement.
(Item 14)
   The classifier of item 13, wherein the predetermined movement comprises a jaw movement, a tongue movement, and a throat movement.
(Item 15)
   The classifier of item 14, wherein the predetermined movement comprises at least a mouth opening and closing motion associated with a motion of the jaw movement, at least a tongue raising motion, a right side translation motion of the tongue, a left side translation motion of the tongue, and a forward extension motion of the tongue associated with a motion of the tongue movement, and at least a dry swallowing motion associated with a motion of the throat movement.
(Item 16)
   The classifier of item 15, wherein the predetermined movement further comprises a tapping motion associated with a motion of the jaw movement and a left and right translation motion of the tongue associated with a motion of the tongue movement.
(Item 17)
   A method for constructing a classifier used for evaluating a movement of a subject, the method comprising:
   detecting a myoelectric signal indicating an activity of a muscle when a subject with no impairment in a predetermined movement comprising a jaw movement, a tongue movement, or a throat movement performs the predetermined movement;
   outputting a processed myoelectric signal by applying predetermined processing on the detected myoelectric signal; and
   learning a pattern contained in the processed myoelectric signal as a pattern in the predetermined movement.
(Item 18)
   A program for constructing a classifier used for evaluating a movement of a subject, the program executed in a computer apparatus comprising a processor unit, wherein the program, when executed, causes the processor unit to perform processing comprising:
   receiving a myoelectric signal indicating an activity of a muscle when a subject with no impairment in a predetermined movement comprising a jaw movement, a tongue movement, or a throat movement performs the predetermined movement;
   outputting a processed myoelectric signal by applying predetermined processing on the detected myoelectric signal; and
   learning a pattern contained in the processed myoelectric signal as a pattern in the predetermined movement.

### [Advantageous Effects of Invention]

The present invention can provide a system, a method, and a program for recognizing myoelectric signal-originating motions comprising at least two of a motion of a jaw movement, a motion of a tongue movement, and a motion of a throat movement.

The present invention can also provide a system, a method, and a program for evaluating a movement of a subject.

### [Brief Description of Drawings]

[Figure 1] Figure 1 is a diagram showing a myoelectric device **100** fitted onto a subject.

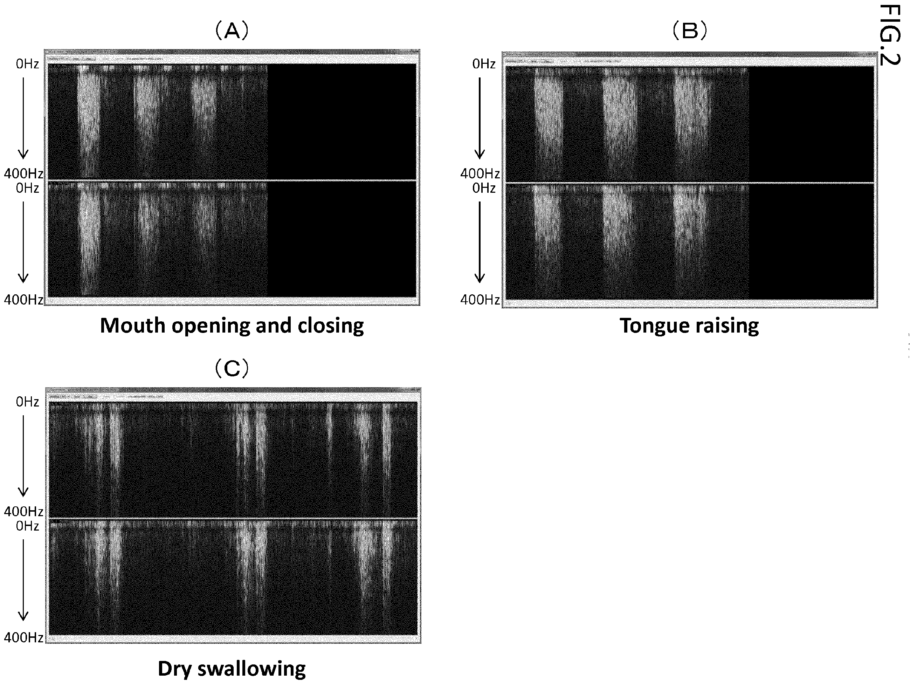

[Figure 2] **(A)** is a graph showing an example of an output after the system of the invention has applied predetermined processing on a myoelectric signal detected by the myoelectric device **100** upon a motion of a jaw movement of a subject. **(B)** is a graph showing an example of an output after the system of the invention has applied predetermined processing on a myoelectric signal detected by the myoelectric device **100** upon a motion of a tongue movement of a subject. **(C)** is a graph showing an example of an output after the system of the invention has applied predetermined processing on a myoelectric signal detected by the myoelectric device **100** upon a motion of a throat movement of a subject.
[Figure 3] Figure **3** is a block diagram depicting an example of a configuration of system **10** for recognizing a myoelectric signal-originating motion.
[Figure 4] Figure **4** is a block diagram depicting an example of a configuration of a computer apparatus **200.**
[Figure 5A] Figure **5A** is a flow chart depicting an example of processing for recognizing a myoelectric signal-originating motion.
[Figure 5B] Figure **5B** is a flow chart depicting an example of processing **510** for evaluating a movement of a subject.
[Figure 6-1] **(A)**-**(B)** are graphs showing an example of an output after frequency analysis processing by processing means **221** in step **S502.**
[Figure 6-2] **(C)-(F)** are graphs showing an example of an output after frequency analysis processing by processing means **221** in step **S502.**
[Figure 6-3] **(G)**-**(J)** are graphs showing an example of an output after frequency analysis processing by processing means **221** in step **S502.**
[Figure 7A] Figure **7A** is a flowchart depicting an example of processing in step **S503** (processing performed by determination means **222** for determining which one or more of a plurality of patterns of motions comprising at least two of a pattern of a motion of a jaw movement, a pattern of a motion of a tongue movement, and a pattern of a motion of a throat movement is a pattern contained in a processed myoelectric signal).
[Figure 7B] Figure **7B** is a flowchart depicting an example of processing in step **S513** (processing performed by determination means **222** for determining a motion level in a jaw movement, a tongue movement, or a throat movement of a subject based on a pattern contained in a processed myoelectric signal).

### [Description of Embodiments]

The embodiments of the invention are explained hereinafter with reference to the drawings.

As used herein, "about" means that the subsequent number is within the range of the number ± 10%.

### 1. Myoelectric device

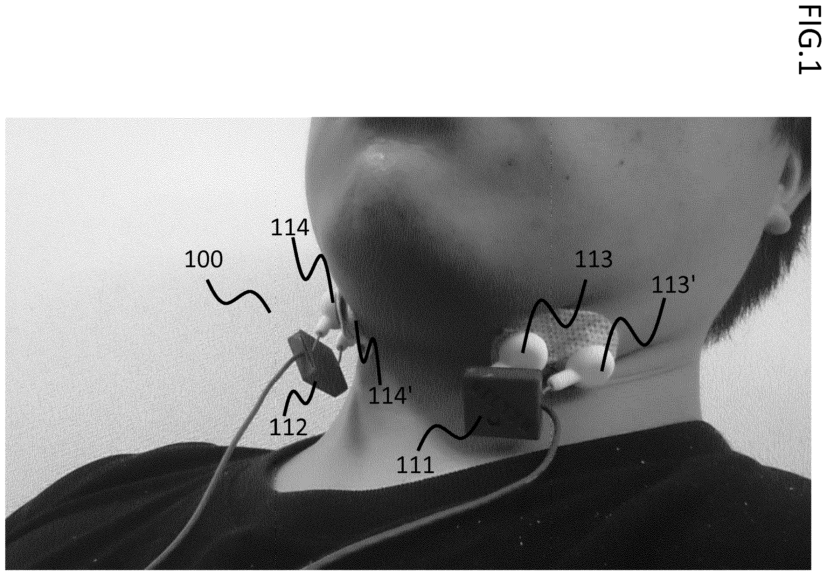

Figure **1** shows a myoelectric device **100** fitted onto a subject.

The myoelectric device **100** comprises a first myoelectric sensor **111** and a second myoelectric sensor **112.** The first myoelectric sensor **111** comprises a pair of measurement electrodes **113, 113'** and a reference electrode (not shown). The second myoelectric sensor **112** comprises a pair of measurement electrodes **114, 114'** and a reference electrode (not shown). The first myoelectric sensor **111** detects a myoelectric signal from an electric signal measured by the pair of measurement electrodes **113, 113'** and the reference electrode. The second myoelectric sensor **112** detects a myoelectric signal from an electric signal measured by the pair of measurement electrodes **114, 114'** and the reference electrode. A myoelectric signal is a signal indicating an activity of a muscle in the body.

The pair of measurement electrodes **113, 113'** and the pair of measurement electrodes **114, 114'** of the myoelectric device **100** are attached to a position on the body of a subject (e.g., position around the throat of the subject) where a myoelectric signal indicating activities of at least two muscles among a jaw muscle, a tongue muscle, and a throat muscle can be detected. For example, the pair of measurement electrodes **113, 113'** is attached to the left side of the Adam's apple, and the pair of measurement electrodes **114, 114'** is attached to the right side of the Adam's apple. The pair of measurement electrodes **113, 113'** and the pair of measurement electrodes **114, 114'** are preferably symmetrical to the left and right with respect to the Adam's apple. The position on the body of a subject where the pair of measurement electrodes **113, 113'** and the pair of measurement electrodes **114, 114'** are attached can be any position, as long as it is a position where a myoelectric signal indicating activities of at least two muscles among a jaw muscle, tongue muscle, and throat muscle can be detected, but is preferably a position where a myoelectric signal indicating activities of a jaw muscle, tongue muscle, and throat muscle can be detected. This is because three motions, i.e., motion of a jaw movement, motion of a tongue movement, and motion of a throat movement, would be identifiable by the processing discussed below. Such a position on the body of a subject can be, for example, the cheek, side of the neck, or the like as well as a position around the throat of the subject.

Reference electrodes of the first myoelectric sensor **111** and the second myoelectric sensor **112** can be the same reference electrode or separate reference electrodes. A reference electrode can be attached to, for example, the back of the neck of the subject.

The first myoelectric sensor **111** can detect a myoelectric signal indicating activities of at least two muscles among a muscle on the left side of the jaw, a muscle of the left side of the tongue, and a muscle of the left side of the throat by a reference electrode and the pair of measurement electrodes **113, 113'** attached to the left side of the throat of the subject. Preferably, the first myoelectric sensor **111** detects a myoelectric signal indicating activities of a muscle on the left side of the jaw, a muscle of the left side of the tongue, and a muscle of the left side of the throat. The second myoelectric sensor **112** can detect a myoelectric signal indicating activities of at least two muscles among a muscle on the right side of the jaw, a muscle of the right side of the tongue, and a muscle of the right side of the throat by a reference electrode and the pair of measurement electrodes **114, 114'** attached to the right side of the throat of the subject. Preferably, the second myoelectric sensor **112** detects a myoelectric signal indicating activities of a muscle on the right side of the jaw, a muscle of the right side of the tongue, and muscle of the right side of the throat.

Myoelectric signals indicating activities of a plurality of muscles coexist in myoelectric signals detected by the myoelectric device **100**. Without any processing, it is impossible to identify the motion from which the myoelectric signal has originated. In this regard, a myoelectric signal detected by the myoelectric device **100** is processed by the system of the invention.

**(A)** in Figure **2** is a graph showing an example of an output after the system of the invention has applied predetermined processing on a myoelectric signal detected by the myoelectric device **100** upon a motion of a jaw movement of a subject. **(B)** in Figure **2** is a graph showing an example of an output after the system of the invention has applied predetermined processing on a myoelectric signal detected by the myoelectric device **100** upon a motion of a tongue movement of a subject. **(C)** in Figure **2** is a graph showing an example of an output after the system of the invention has applied predetermined processing on a myoelectric signal detected by the myoelectric device **100-**upon a motion of a throat movement of a subject.

Examples of the predetermined processing performed by the system of the invention include frequency analysis processing such as short-time FFT. A time series data for a frequency distribution can be obtained with such frequency analysis processing. **(A)** in Figure **2** shows time series data for a frequency distribution obtained from myoelectric signals detected when a subject has opened and closed the mouth (mouth opening and closing motion) three times. **(B)** in Figure **2** shows time series data for a frequency distribution obtained from myoelectric signals detected when a subject has pushed the tongue against the upper jaw (tongue raising motion) three times. **(C)** in Figure **2** shows time series data for a frequency distribution obtained from myoelectric signals detected when a subject has swallowed without anything in the mouth (dry swallowing motion) three times. In **(A)** to **(C)** of Figures **2****,** the horizontal axis represents the time, and the vertical axis represents frequency. The top of the vertical axis is 0 Hz, and the bottom of the vertical axis is 400 Hz. The color on the graph represents the intensity of frequency components. Darker color indicates weaker frequency component, and lighter color indicates stronger frequency component. In each of **(A)** to **(C)** in Figures **2****,** the graph on the top side shows data from an output of the myoelectric sensor **111** attached to the left side of the throat of the subject, and the graph on the bottom side shows data from an output of the myoelectric sensor **112** attached to the right side of the throat of the subject.

The frequency distributions of myoelectric signals in **(A)** to **(C)** in Figures **2** are compared. For the mouth opening and closing motion, frequencies of myoelectric signals are evenly distributed, especially strongly at about 50 to about 200 Hz, while widely distributed throughout the entire frequency band of myoelectric signals. For the tongue raising motion, frequencies of myoelectric signals are distributed strongly at about 50 to 200 Hz and weakly at high frequency bands above about 200 Hz, with about 100 Hz as the center. For the dry swallowing motion, frequencies of myoelectric signals are distributed especially strongly at about 100 Hz and weakly at high frequency bands above about 150 Hz.

The changes in time series of the frequency distributions of myoelectric signals in **(A)** to **(C)** in Figures **2** are compared. For the mouth opening and closing motion, the frequency decreases over time. For the tongue raising motion, the frequency tends to not decrease over time. For the dry swallowing motion, there are two large peaks.

In this manner, an output after the system of the invention has applied predetermined processing on myoelectric signals detected by the myoelectric device **100** contains a specific pattern that can be matched to at least a motion of a jaw movement, a motion of a tongue movement, or a motion of a throat movement. The detected myoelectric signal-originating motion can be recognized as a motion of a jaw movement, a motion of a tongue movement, or a motion of a throat movement by recognizing these patterns.

Recognition of whether the detected myoelectric signal-originating motion is a motion of a jaw movement, a motion of a tongue movement, or a motion of a throat movement can be utilized in, for example, evaluation or rehabilitation of a motor function for swallowing or chewing.

For example, the system of the invention can determine the motion level in a jaw movement, tongue movement, or throat movement by learning the patterns of a motion of a jaw movement, a motion of a tongue movement, and a motion of a throat movement of a large number of healthy individuals as supervisory patterns and utilizing the result of matching the patterns of a motion of a jaw movement, a motion of a tongue movement, and a motion of a throat movement of a subject with the supervisory patterns. Determination of a motion level can quantitatively determine the size of a motion performed by a subject based on the size of motion of healthy individuals, or qualitatively determine whether the motion performed by the subject is normal. Medical practitioners such as physicians and rehab advisers can ultimately evaluate, judge, and determine whether the motor function for swallowing or chewing of a subject is normal by using an output from the system of the invention as an indicator.

For example, the system of the invention can quantify the degree of motor dysfunction or impairment of the jaw, tongue, or throat by utilizing the result of matching the patterns of a motion of a jaw movement, a motion of a tongue movement, and a motion of a throat movement of a subject with supervisory patterns, based on a detected myoelectric signal. Medical practitioners (physicians, rehab advisers, etc.) can provide appropriate guidance for rehabilitation that is in alignment with the site and degree of motor dysfunction or impairment by using an output from the system of the invention as an indicator. For example, a detected myoelectric signal can be utilized as an indicator to evaluate whether a patient can perform a motion properly by administering training to a patient for a specific motion among a jaw movement, tongue movement, and throat movement for rehabilitation. To show whether a subject is able to perform a motion properly, an avatar of the subject can be displayed on a monitor and make the avatar of the subject express the extent of motion based on the detected myoelectric signal. This enables the subjects to objectively see the actual extent of the motion intended by themselves.

For example, the precision of determining the motion level in a jaw movement, tongue movement, or throat movement can be improved by learning patterns of a motion of a jaw movement, a motion of a tongue movement, and a motion of a throat movement of a large number of healthy individuals as supervisory patterns. However, it should be noted that evaluation of a motor function for swallowing or chewing of a subject by medical practitioners discussed above is intended to assist the medical practitioners, so that the precision of determining a motion level can be somewhat low (e.g., 60%, 70%, or the like).

### 2. Configuration of a system for recognizing myoelectric signal-originating motion

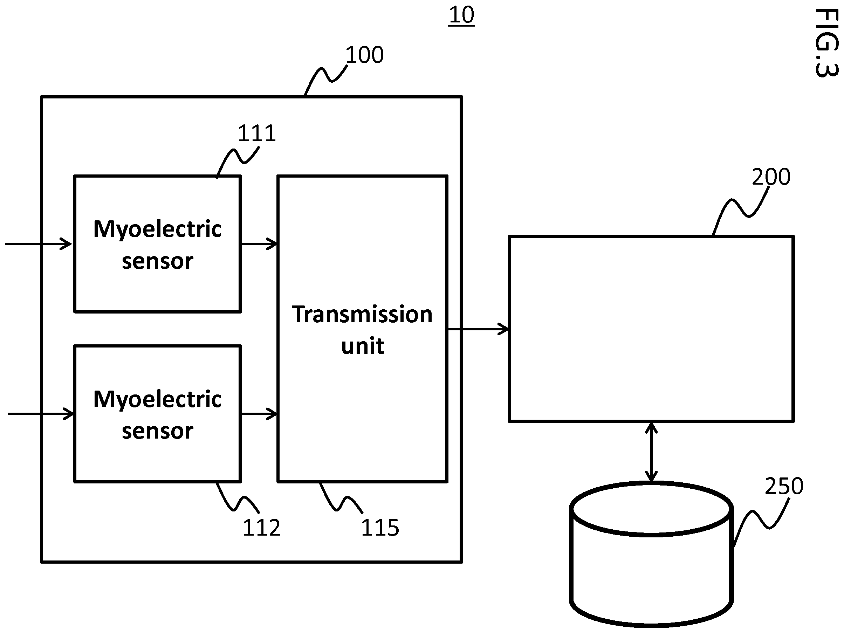

Figure **3** depicts an example of a configuration of system **10** for recognizing a myoelectric signal-originating motion. In this regard, myoelectric signal-originating motions comprise at least two of a motion of a jaw movement, a motion of a tongue movement, and a motion of a throat movement, but preferably comprises three motions, i.e., a motion of a jaw movement, a motion of a tongue movement, and a motion of a throat movement.

The system **10** comprises a myoelectric device **100** and a computer apparatus **200.** A database unit **250** is connected to the computer apparatus **200.** The myoelectric device **100** and the computer apparatus **200** are connected via a network. In this regard, the network can be any type of network. For example, the myoelectric device **100** and the computer apparatus **200** can communicate with each other via the Internet or via LAN.

The myoelectric device **100** comprises two myoelectric sensors **111, 112** and a transmission unit **115.**

The myoelectric sensor **111** comprises a pair of measurement electrodes **113, 113'** and a reference electrode, as shown in Figure **1****.** The myoelectric sensor **112** comprises a pair of measurement electrodes **114, 114'** and a reference electrode, as shown in Figure **1****.** The myoelectric sensors **111, 112** can be any detection means that is capable of detecting a myoelectric signal indicating the activity of a muscle in the body. For example, the myoelectric sensors **111, 112** can comprise a primary amplifier, a high pass filter, a low pass filter, a notch filter, and a secondary amplifier for the detection of myoelectric signals. Primary and secondary amplifiers are used for amplifying a signal. A high pass filter is used for attenuating a signal with a frequency lower than a predetermined frequency, e.g., a signal with a frequency lower than about 10 Hz. A low pass filter is used for attenuating a signal with a frequency higher than a predetermined frequency, e.g., a signal with a frequency higher than about 400 Hz. A notch filter is used to attenuate a signal with a frequency in a predetermined range, e.g., 50 to 60 Hz AC noise, which is a typical electrical noise. A band elimination filter can also be used in place of a notch filter.

The transmission unit **115** is configured to transmit a signal out of the myoelectric device **100**. The transmission unit **115** transmits a signal out of the myoelectric device **100** by wireless or wired transmission. For example, the transmission unit **115** can transmit a signal by utilizing a wireless LAN such as Wi-Fi. For example, the transmission unit **115** can transmit a signal by utilizing a short range wireless communication such as Bluetooth®. The transmission unit **115** transmits a myoelectric signal indicating activities of a plurality of muscles in the body including a jaw muscle, tongue muscle, and throat muscle detected by a myoelectric sensor to the computer apparatus **200.**

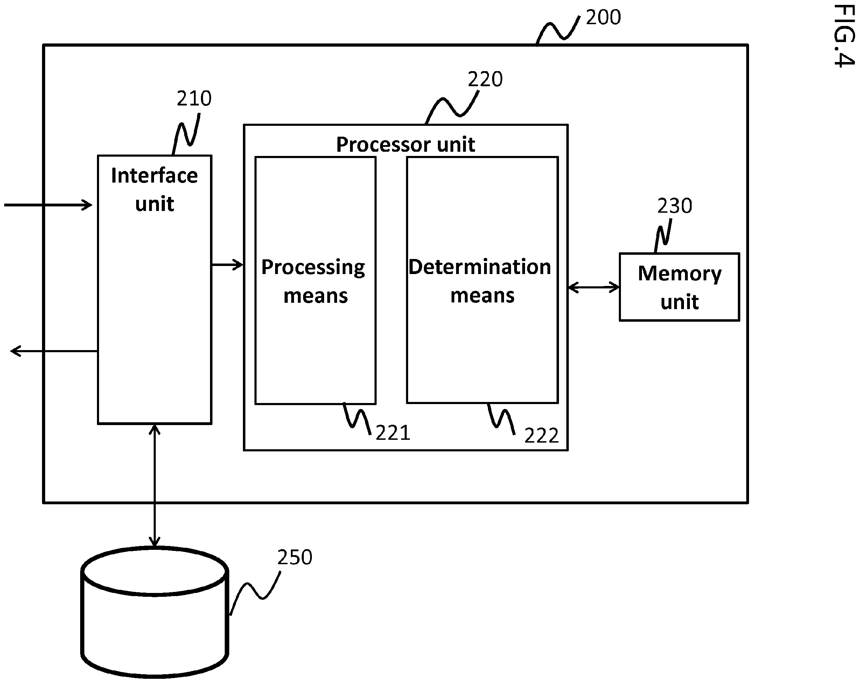

Figure **4** depicts an example of a configuration of the computer apparatus **200.**

The computer apparatus **200** comprises an interface unit **210,** a processor unit **220,** and a memory unit **230.** As discussed above, the database unit **250** is connected to the computer apparatus **200.**

The interface unit **210** is configured to control communication of the computer apparatus **200** with the outside. The interface unit **210** can transmit information out of the computer apparatus **200** and receive information from the outside of the computer apparatus **200.** For example, the interface unit **210** controls communication with the myoelectric device **100** or the database unit **250.** The interface unit **210** can control communication by any method. Communication can be wired communication or wireless communication.

The processor unit **220** controls the operation of the entire computer apparatus **200.** The processor unit **220** reads out a program stored in the memory unit **230** and executes the program. This allows the computer apparatus **200** to function as an apparatus that executes desired steps.

The processor unit **220** comprises processing means **221** and determination means **222.**

The processing means **221** is configured to output a processed myoelectric signal by applying predetermined processing on an input myoelectric signal. Predetermined processing comprises, for example, signal waveform drawing processing, signal intensity extraction processing, and frequency analysis processing. Signal waveform drawing processing is processing for drawing a waveform of a detected myoelectric signal in time series. The waveform itself of a myoelectric signal can be studied from such an output. Signal intensity extraction processing is processing for extracting the intensity of a detected myoelectric signal to obtain time series data. The intensity of a myoelectric signal is a value that is proportional to the force exerted by a myoelectric signal-originating muscle. Signal intensity extraction processing can be, for example, a combination of full-wave rectification processing and moving average processing. Frequency analysis processing is processing for obtaining time series data for a frequency distribution of a detected myoelectric signal. Since the frequency distribution of myoelectric signals varies by muscle, differences in the primary active muscle can be observed by performing frequency analysis processing. With frequency analysis processing, muscle activities can also be observed even if the myoelectric signal intensity is minute. Frequency analysis processing comprises, for example, short-time FFT. Short-time FFT applies FFT on a myoelectric signal obtained in any short period of time (e.g., about 0.01 seconds to about 1 second, preferably about 0.1 seconds or about 128/1600 seconds). Shorter time interval leads to higher response rate (rate of an output responding to an input), while leading to lower frequency resolution, so that stability of a signal is not guaranteed. In contrast, longer time interval (e.g., about 256/1600 seconds or longer) leads to higher frequency resolution, but a lower response rate. Thus, the precision in time decreases, so that evaluation in real time would not be possible. Longer time interval also increases the amount of calculation, resulting in higher hardware cost. For example, if the time interval is about 0.1 seconds, the response rate and frequency resolution can be maintained to the extent that a pattern contained in a myoelectric signal can identify a pattern of a motion of a jaw movement, a pattern of a motion of a tongue movement, or a pattern of a motion of a throat movement, so that signal stability and real-time evaluation can both be attained with relatively low amount of calculation. For example, FFT is performed at an interval of about 0.1 seconds, i.e., FFT is applied on a myoelectric signal obtained at 0 to about 0.1 seconds, FFT is applied on a myoelectric signal obtained at about 0.1 to about 0.2 seconds, FFT is applied on a myoelectric signal obtained at about 0.2 to about 0.3 seconds ... FFT is applied on a myoelectric signal obtained at (n - 0.1) to n seconds. Time series data for a frequency distribution can be obtained by connecting a plurality of frequency distributions obtained by short-time FFT.

The determination means **222** is configured to determine which one or more of a plurality of patterns of motions comprising at least two of a pattern of a motion of a jaw movement, a pattern of a motion of a tongue movement, and a motion of a throat movement is a pattern contained in the processed myoelectric signal outputted by the processing means **221.** The determination means **222** determines which one or more of a plurality of patterns of motions comprising at least two of a pattern of a motion of a jaw movement, a pattern of a motion of a tongue movement, and pattern of a motion of a throat movement is a pattern contained in the processed myoelectric signal by, for example, matching a pattern with a supervisory pattern of a motion stored in the database unit **250.** In this regard, the determination means **222** can be configured to comprise learning means for learning at least two of a supervisory pattern of a motion of a jaw movement, a supervisory pattern of a motion of a tongue movement, and a supervisory pattern of a motion of a throat movement, calculation means for calculating a degree of similarity between a processed myoelectric signal and the supervisory pattern, and judgment means for judging whether the degree of similarity exceeds a predetermined threshold value. The learning means can also learn a supervisory pattern for no motion being performed in addition to a supervisory pattern of a motion of a jaw movement, a supervisory pattern of a motion of a tongue movement, and a supervisory pattern of a motion of a throat movement. This is because even if a subject is not performing a motion, a myoelectric signal is not necessarily zero due to a force that is unconsciously exerted, effect of environmental noise, or the like. This enables the determination means **222** to determine that a pattern contained in a processed myoelectric signal is a pattern for no motion being performed.

The determination means **222** can determine which one of the plurality of patterns of motions is each of the plurality of patterns contained in a processed myoelectric signal, even when the processed myoelectric signal contains a plurality of patterns. This enables recognition of such motions even if a myoelectric signal originates from a composite motion.

The determination means **222** can be configured to determine a motion level or evaluate the presence or absence of an impairment in a jaw movement, tongue movement, or throat movement of a subject, based on a pattern contained in a processed myoelectric signal outputted from the processing means **221,** in addition to, or instead of, the aforementioned configuration. For example, the determination means **222** determines a motion level or evaluates the presence or absence of an impairment in a jaw movement, tongue movement, or throat movement of a subject, based on a pattern contained in a processed myoelectric signal by matching a pattern with a supervisory pattern of motion stored in the database unit **250.** In this regard, the determination means **222** can be configured to comprise learning means for learning a supervisory pattern of a motion of a jaw movement, a supervisory pattern of a motion of a tongue movement, or a supervisory pattern of a motion of a throat movement, and calculation means for calculating a degree of similarity between a processed myoelectric signal and the supervisory pattern. The determination means **222** can be configured to further comprise judgment means for judging whether the degree of similarity exceeds a predetermined threshold value. For example, the determination means **222** can determine that there is an impairment in a jaw movement, tongue movement, or throat movement of a subject if the degree of similarity does not exceed a predetermined threshold value. The system **10** comprising such determination means **222** can be used to evaluate a movement of a subject.

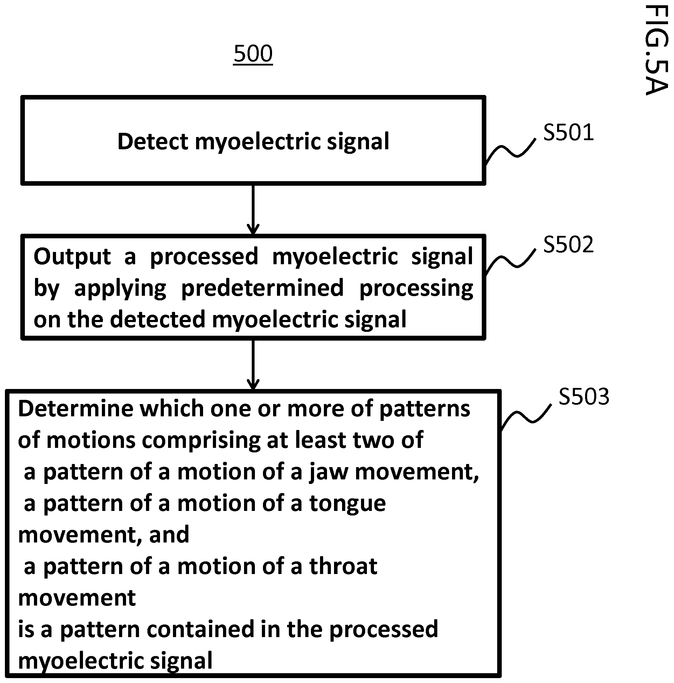

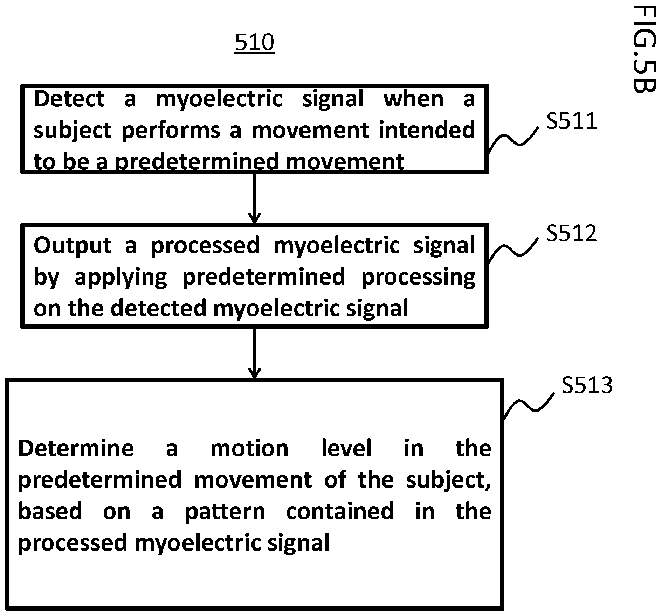

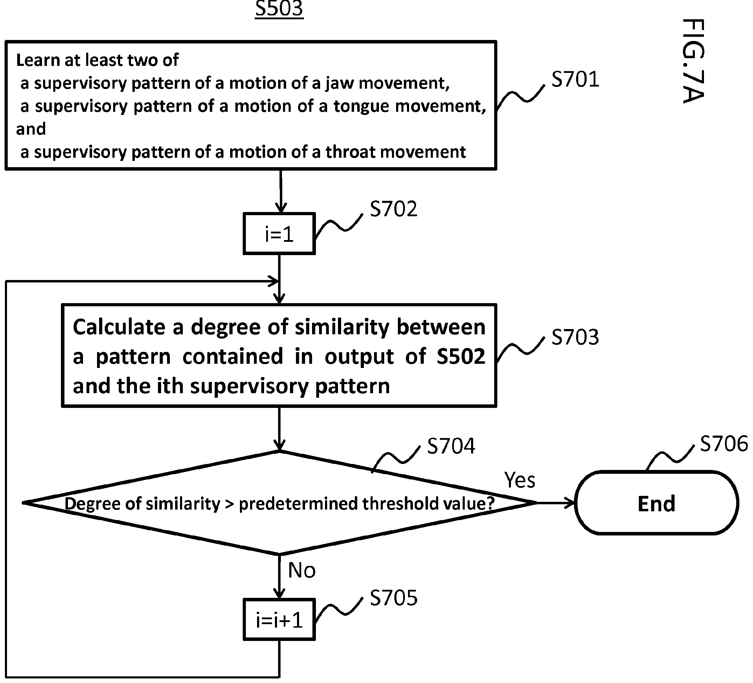

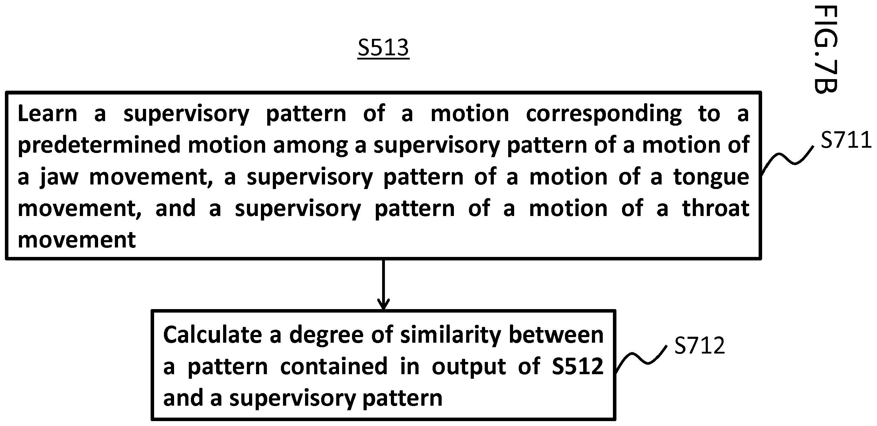

The memory unit **230** stores a program required to execute processing, data required to execute the program, and the like. For example, a program for materializing processing for recognizing a myoelectric signal-originating motion (e.g., processing discussed below in Figures **5A** and **7A**), processing for evaluating a movement of a subject (e.g., processing discussed below in Figures **5B** and **7B**), or processing for constructing a classifier used for evaluating a movement of a subject can be stored in the memory unit **230.** In this regard, the program can be stored in the memory unit **230** by any manner. For example, a program can be preinstalled in the memory unit **230.** Alternatively, a program can be installed in the memory unit **230** by downloading the program through a network, or installed in the memory unit **230** via a storage medium such as an optical disk or USB.

For example, a pattern of a motion of a jaw movement, a pattern of a motion of a tongue movement, a pattern of a motion of a throat movement, or a pattern for no motion being performed recognized from a myoelectric signal transmitted from the myoelectric device **100** can be stored in the database unit **250** while being associated with a user of the myoelectric device **100**. A supervisory pattern of a motion of a jaw movement, a supervisory pattern of a motion of a tongue movement, a supervisory pattern of a motion of a throat movement, or a supervisory pattern for no motion being performed learned by learning means of the determination means **222** can also be stored in the database unit **250.** The database unit **250** can be configured to store a weighting coefficient for each node of a neural network discussed below.

The example depicted in Figure **3** has explained an example of the myoelectric device **100** comprising two myoelectric sensors 111, 112, but the number of myoelectric sensors is not limited thereto. The myoelectric device **100** comprising three or more myoelectric sensors is also within the scope of the present invention.

While each constituent element of the computer apparatus **200** is provided within the computer apparatus **200** in the examples depicted in Figures **3** and **4****,** the present invention is not limited thereto. Any of the constituent elements of the computer apparatus **200** can be provided outside of the computer apparatus **200.** For example, if each of the processor unit **220** and the memory unit **230** is comprised of separate hardware parts, each hardware part can be connected via any network. In this regard, the network can be any type of network. For example, each hardware part can be connected via LAN, wireless connection, or wired connection.

In the examples depicted in Figures **3** and **4****,** the database unit **250** is provided external to the computer apparatus **200,** but the present invention is not limited thereto. The database unit **250** can be provided inside the computer apparatus **200.** In this regard, the database unit **250** can be implemented by the same storing means as the storing means implementing the memory unit **230,** or by storage means that is different from the storing means implementing the memory unit **230.** In either case, the database unit **250** is configured as a storing unit for the computer apparatus **200.** The configuration of the database unit **250** is not limited to a specific hardware configuration. For example, the database unit **250** can be configured with a single hardware part or a plurality of hardware parts. For example, the database unit **250** can be configured as an external hard disk apparatus of the computer apparatus **200** or as a storage on the cloud connected via a network.

### 3. Processing for recognizing myoelectric signal-originating motion

Figure **5A** depicts an example of processing **500** for recognizing a myoelectric signal-originating motion. This processing is executed by the system **10**. In this regard, a myoelectric signal-originating motion comprises at least two of a motion of a jaw movement, a motion of a tongue movement, and a motion of a throat movement, but preferably comprises three motions, i.e., a motion of a jaw movement, a motion of a tongue movement, and a motion of a throat movement.

In step **S501**, the first myoelectric sensor **111** and the second myoelectric sensor **112** of the myoelectric device **100** detect a myoelectric signal indicating activities of a plurality of muscles. The first myoelectric sensor **111** detects a myoelectric signal using a pair of measurement electrodes **113, 113**' and a reference electrode. The second myoelectric sensor **112** detects a myoelectric signal using a pair of measurement electrodes **114, 114'** and a reference electrode. If such myoelectric signals are detected, the myoelectric device **100** transmits the detected myoelectric signals to the computer apparatus **200** via the transmission unit **115.** The computer apparatus **200** receives the detected myoelectric signals via the interface unit **210.**

In step **S502**, the processing means **221** of the processor unit **220** of the computer apparatus **200** applies predetermined processing on a myoelectric signal detected by the myoelectric device **100** to output a processed myoelectric signal. The predetermined processing comprises, for example, signal waveform drawing processing, signal intensity extraction processing, and frequency analysis processing. The processed myoelectric signal outputted from the processing means **221** is provided to the determination means **222.**

In step **S503**, the determination means **222** of the processor unit **220** determines which one or more of patterns of motions comprising at least two of a pattern of a motion of a jaw movement, a pattern of a motion of a tongue movement, and a pattern of a motion of a throat movement is a pattern contained in the processed myoelectric signal. A processed myoelectric signal comprises a specific pattern that can be matched at least to a motion of a jaw movement, a motion of a tongue movement, or a motion of a throat movement. The determination means **222** determines which one or more of patterns of motions comprising at least two of a pattern of a motion of a jaw movement, a pattern of a motion of a tongue movement, and a pattern of a motion of a throat movement is this pattern. For example, the determination means **222** determines which one or more of patterns of motions comprising at least two of a pattern of a motion of a jaw movement, a pattern of a motion of a tongue movement, and a pattern of a motion of a throat movement is a pattern contained in the processed myoelectric signal by the processing discussed below in Figure **7A****.** In this regard, the determination means **222** can also determine that the pattern contained in the processed myoelectric signal is a pattern for no motion being performed. Alternatively, the determination means **222** can also determine that the patterns contained in the processed myoelectric signal are patterns for a plurality of motions, and determine which pattern corresponds to each of the patterns of the plurality of motions.

It is possible to recognize, with the processing discussed above, whether a myoelectric signal-originating motion is a motion of a jaw movement, a motion of a tongue movement, a motion of a throat movement, or a composite motion.

Since it is possible to recognize whether a myoelectric signal-originating motion is a motion of a jaw movement, a motion of a tongue movement, a motion of a throat movement, or a composite motion, the system **10** of the invention can, for example, evaluate whether there is an impairment in a movement of a subject or not, or evaluate a motion level of a movement of a subject based thereon.

Figure **5B** depicts an example of processing **510** for evaluating a movement of a subject. This processing is executed in the system **10**.

In step **S511,** the first myoelectric sensor **111** and the second myoelectric sensor **112** of the myoelectric device **100** detect a myoelectric signal indicating an activity of a muscle when a subject performs a movement intending a predetermined movement comprising a jaw movement, a tongue movement, or a throat movement. Since this step is the same as step **S501** of processing **500,** the explanation is omitted.

In step **S512**, the processing means **221** of the processor unit **220** of the computer apparatus **200** outputs a processed myoelectric signal by applying predetermined processing on a myoelectric signal detected by the myoelectric device **100.** Since this step is the same as step **S502** of processing **500,** the explanation is omitted.

In step **S513**, the determination means **222** of the processor unit **220** determines a motion level in a predetermined movement of a subject based on a pattern contained in a processed myoelectric signal. The processed myoelectric signal at least comprises a specific pattern that can be matched to a motion of a jaw movement of a healthy individual, a motion of a tongue movement of a healthy individual, or a motion of a throat movement of a healthy individual. The determination means **222** determines a motion level in a predetermined movement comprising a jaw movement, a tongue movement, or a throat movement of a subject based on such patterns. The determination means **222,** for example, calculates the degree of similarity between a pattern contained in a processed myoelectric signal and a pattern of a motion of a jaw movement of a healthy individual, a pattern of a motion of a tongue movement of a healthy individual, or pattern of a motion of a throat movement of a healthy individual by the processing discussed below in Figure **7B** and determines a motion level in a predetermined movement comprising a jaw movement, tongue movement, or throat movement of a subject based on the degree of similarity. For example, the determination means **222** can be configured to evaluate the presence or absence of an impairment in a predetermined movement comprising a jaw movement, tongue movement, or throat movement of a subject based on the degree of similarity.

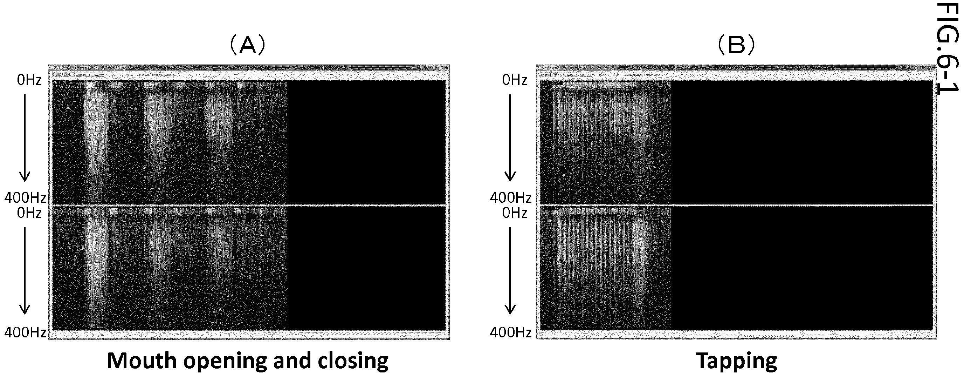

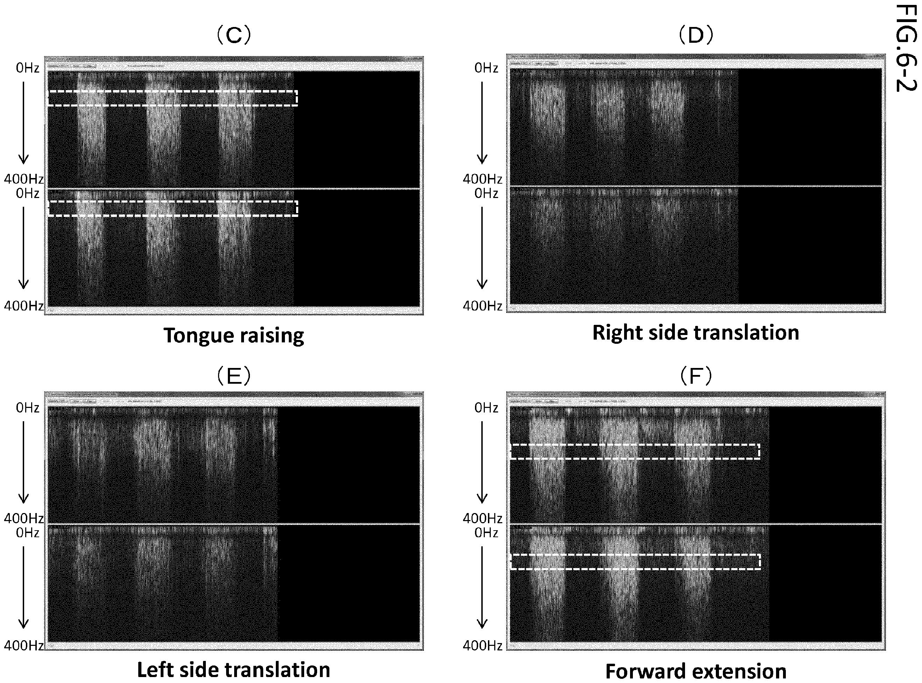

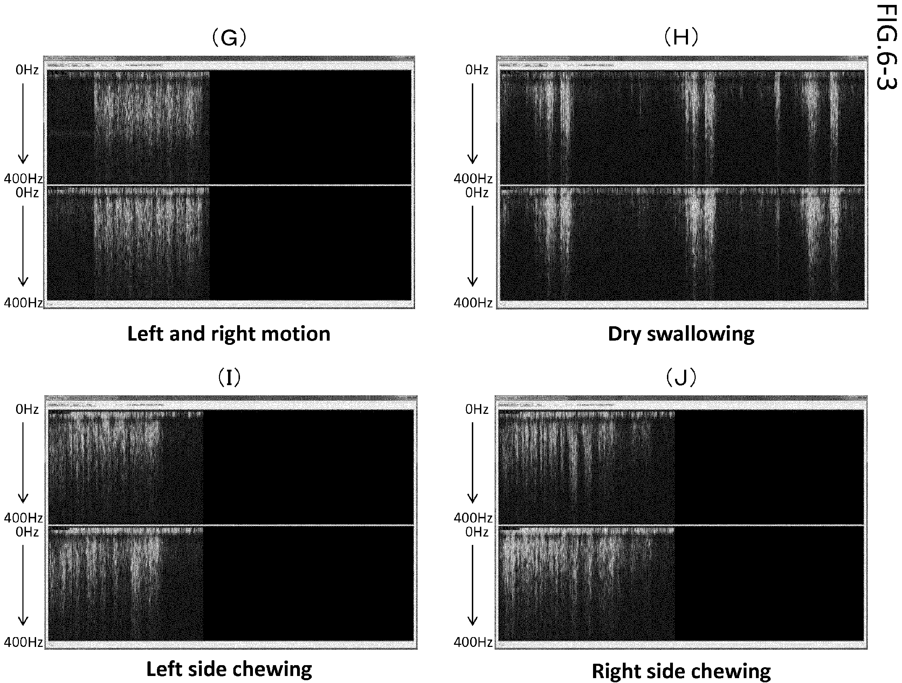

**(A)** to **(J)** in Figure **6** are graphs showing an example of an output after frequency analysis processing by processing means **221** in step **S502**. In **(A)** to **(J)** in Figure **6****,** the horizontal axis represents time, and the vertical axis represents frequency in the same manner as **(A)** to **(C)** in Figure **2****.** The top of the vertical axis is 0 Hz, and the bottom of the vertical axis is about 400 Hz. The color on the graphs represents the intensity of frequency components. Darker color indicates weaker frequency component, and lighter color indicates stronger frequency component. In each of **(A)** to **(J)** in Figure **6****,** the graph on the top side shows data from an output of the myoelectric sensor 111 attached to the left side of the throat of the subject, and the graph on the bottom side shows data from an output of the myoelectric sensor **112** attached to the right side of the throat of the subject.
**(A)** in Figure **6** shows time series data for a frequency distribution obtained from myoelectric signals detected when a subject has opened and closed the mouth (mouth opening and closing motion) three times. For the mouth opening and closing motion, frequencies of myoelectric signals are evenly distributed, especially strongly at about 50 to about 200 Hz, while widely distributed throughout the entire frequency band of myoelectric signals of about 10 to about 400 Hz.
**(B)** in Figure **6** shows time series data for a frequency distribution obtained from myoelectric signals detected when a subject has consecutively struck the top and bottom teeth against each other (tapping motion). For the tapping motion, frequencies of myoelectric signals are evenly distributed, especially strongly at about 50 to about 200 Hz, while widely distributed throughout the entire frequency band of myoelectric signals of about 10 to about 400 Hz. For a tapping motion, the frequency distribution and signal intensity vary at the same cycle as tapping (e.g., 0.1 seconds, 0.2 seconds, 0.3 seconds, 0.5 seconds, etc.)
**(C)** in Figure **6** shows time series data for a frequency distribution obtained from myoelectric signals detected when a subject has pushed the tongue against the upper jaw (tongue raising motion) three times. For the tongue raising motion, frequencies of myoelectric signals are distributed strongly at about 50 to 200 Hz and weakly at high frequency bands above about 200 Hz, with about 100 Hz as the center.
**(D)** in Figure **6** shows time series data for a frequency distribution obtained from myoelectric signals detected when a subject has translated the tongue to the right side (right side translation motion) three times. For the right side translation motion, frequencies of myoelectric signals are distributed strongly at about 50 to 200 Hz and weakly at high frequency bands above about 200 Hz, with about 100 Hz as the center.
**(E)** in Figure **6** shows time series data for a frequency distribution obtained from myoelectric signals detected when a subject has translated the tongue to the left side (left side translation motion) three times. For the left side translation motion, frequencies of myoelectric signals are distributed strongly at about 50 to 200 Hz and weakly at high frequency bands above about 200 Hz, with about 100 Hz as the center.
**(F)** in Figure **6** shows time series data for a frequency distribution obtained from myoelectric signals detected when a subject has pushed the tongue forward (forward extension motion) three times. For the forward extension motion, frequencies of myoelectric signals are distributed to be high around about 150 Hz.
**(G)** in Figure **6** shows time series data for a frequency distribution obtained from myoelectric signals detected when a subject has consecutively translated the tongue to the left and right (left and right movement motion) three times. For the left and right motion, frequencies of myoelectric signals are distributed strongly at about 50 to 200 Hz and weakly at high frequency bands above about 200 Hz, with about 100 Hz as the center. For the left and right motion, the frequency distribution and signal intensity vary at the same cycle as the left and right motion (e.g., about 0.5 seconds, about 1 second, etc.)
**(H)** in Figure **6** shows time series data for a frequency distribution obtained from myoelectric signals detected when a subject has swallowed without anything in the mouth (dry swallowing motion) three times. For the dry swallowing motion, frequencies of myoelectric signals are distributed especially strongly at about 100 Hz and weakly at high frequency bands above about 150 Hz. For the dry swallowing motion, the frequency and signal intensity vary so that there are two consecutive peaks in the time series.
**(I)** in Figure **6** shows time series data for a frequency distribution obtained from myoelectric signals detected when a subject has chewed a chew managing food on the left side (left side chewing motion). For the left side chewing motion, frequencies of myoelectric signals are distributed weakly at high frequency bands above about 200 Hz, with about 100 Hz as the center. For the left side chewing motion, the frequency distribution and signal intensity vary at the same cycle as the chewing (e.g., about 0.1 seconds, about 0.2 seconds, about 0.3 seconds, about 0.5 seconds, etc.)
**(J)** in Figure **6** shows time series data for a frequency distribution obtained from myoelectric signals detected when a subject has chewed a chew managing food on the right side (right side chewing motion). For the right side chewing motion, frequencies of myoelectric signals are distributed weakly at high frequency bands above about 200 Hz, with about 100 Hz as the center. For the right side chewing motion, the frequency distribution and signal intensity vary at the same cycle as the chewing (e.g., about 0.1 seconds, about 0.2 seconds, about 0.3 seconds, about 0.5 seconds, etc.)

**(A)** in Figure **6** shows the same data as **(A)** in Figure **2****, (C)** in Figure **6** shows the same data as **(B)** in Figure **2****,** and **(H)** in Figure **6** shows the same data as **(C)** in Figure **2****.**

As discussed above in reference to **(A)** to **(C)** in Figure **2****,** a mouth opening and closing motion, a tongue raising motion, and a dry swallowing motion can be distinguished, for example, by the frequency distribution of myoelectric signals and the change in time series thereof.

For example, it can be understood by comparing the intensities of frequency components in **(C)** to **(E)** in Figure **6** that the intensities of myoelectric signals in tongue raising motions and forward extension motions are higher than intensities of myoelectric signals in right side translation motions and left side translation motions. In this manner, a tongue raising motion and a forward extension motion can be distinguished from a right side translation motion and a left side translation motion by comparing the intensities of frequency components.

For example, it can be understood by comparing the frequency distributions in **(C)** and **(E)** in Figure **6** that the band with a strong frequency component of a myoelectric signal in a tongue raising motion is in a low frequency band in the vicinity of 100 Hz (band surrounded by a dotted line in **(C)** in Figure **6**), while the band with a strong frequency component of a myoelectric signal in a forward extension motion is in a frequency band greater than 100 Hz (band surrounded by a dotted line in **(F)** in Figure **6**). In this manner, a tongue raising motion and a forward extension motion can be distinguished by comparing frequency distributions

For example, in view of comparing the changes in time series of the frequency distributions in **(B), (I),** and **(J)** in Figure **6****,** a tapping motion is the same as a left side chewing motion and a right side chewing motion in that a striped pattern of short intervals due to a jaw movement can be observed. However, since the tongue or throat muscle is also active in addition to the jaw muscle in a chewing motion, there are myoelectric signals due to a tongue or throat movement between the stripe patterns.

Table 1 summarizes examples of a characteristic of a signal intensity distribution, a characteristic of a frequency distribution, and a characteristic of time series of a pattern of a motion of a jaw movement, a pattern of a motion of a tongue movement, and a pattern of a motion of a throat movement obtained from time series data for frequency distributions shown in **(A)** to **(H)** of Figure **6** and corresponding signal intensity distributions.

**[Table 1]**

| Movement | Motion | Characteristic of intensity distribution | Characteristic of frequency distribution | Characteristic of time series |
|---|---|---|---|---|
| Jaw movement | Mouth opening and closing | Signal intensity is stronger relative to tongue movement | Evenly distributed, especially strongly near 50 to 200 Hz, while widely distributed throughout the entire myoelectric frequency band | Decease over time |
| | Tapping | Same as above | Same as above | Change in much shorter intervals relative to mouth opening and closing |
| Tongue movement | Tongue raising | Signal intensity is | Distributed strongly near | Tend not to decrease over |
| | | weaker relative to jaw movement | 50 to 200 Hz and weakly at high frequency bands above 200 Hz, with around 100 Hz as the center | time |
| | Left side translation | Signal intensity is weaker overall relative to tongue raising; left-right balance is right > left | Same as above | Same as above |
| | Right side translation | Signal intensity is weaker overall relative to tongue raising; left-right balance is left > right | Same as above | Same as above |
| | Left and right movement | Alternating movement to left and right | Same as left | Same as left |
| | Forward extension | Have characteristics of both tongue raising and mouth opening and closing | Distributed strongly in frequency band higher than tongue raising | Have characteristics of both tongue raising and mouth opening and closing |
| Throat movement | Dry swallowing | Signal intensity is greatest relative to other movements | Especially strong near 100 Hz, but dramatically weaker above 150 Hz | Have two large peaks, and space between two peaks are almost the same |

As can be understood from Table 1, the signal intensity of a throat movement is the strongest, the signal intensity of a jaw movement is the second strongest, and the signal intensity of a tongue movement is the weakest. This is due to the following reasons. Since two pairs of measurement electrodes (**113**, **113**') (**114**, **114**') of the myoelectric sensors **11, 12** are attached around the throat, the myoelectric sensors **11, 12** more strongly detect myoelectric signals indicating activities of a throat muscle, and a throat muscle is the largest muscle among jaw muscle, tongue muscle, and throat muscle. In other words, the pattern of signal intensities can vary depending on the measurement position of the two pairs of measurement electrodes **(113, 113**') (**114, 114'**).

For example, if the signal intensity distribution obtained from myoelectric signals detected from a subject chewing a chew managing food 5 times on one side (5 times chewing motion) is compared with the signal intensity distribution obtained from myoelectric signals detected from a subject chewing a chew managing food freely for 10 seconds (10 second chewing motion), the signal intensity from a 5 time chewing motion is stronger than the signal intensity from a 10 second chewing motion. This is because reduction of the number of chewing leads to forced swallowing, which puts a burden on a throat muscle.

The motion patterns discussed above are patterns observed mainly in healthy adults. Different motion patterns are exhibited in healthy children in some cases. For example, two consecutive peaks were not observed in the time series observed in healthy adults, but instead only one peak was observed in some cases in myoelectric signals obtained from children with respect to the change in time series of frequencies in a dry swallowing motion. This is understood to be because muscles of children are undeveloped so that only the muscle of one of the tongue and throat is used for the swallowing motion. It can be determined whether an adult pattern is observed or a child pattern is observed based on, for example, secondary sexual characteristics. For example, an adult pattern is observed in subjects who have developed secondary sexual characteristics, and a child pattern is observed in subjects who have yet to develop secondary sexual characteristics.

For example, the system **10** can recognize whether a myoelectric signal-originating motion of a subject is a motion of a jaw movement, a motion of a tongue movement, a motion of a throat movement, or a composite motion regardless of whether the subject is an adult or a child by switching between an operational mode for identifying an adult pattern and an operational mode for identifying a child pattern. Such an output result from the system **10** can be utilized in evaluation or rehabilitation of a motor function for swallowing/chewing in both adults and children.

Figure **7A** depicts an example of processing in step **S503** (processing performed by determination means **222** for determining which one or more of patterns of motions comprising at least two of a pattern of a motion of a jaw movement, a pattern of a motion of a tongue movement, and a pattern of a motion of a throat movement correspond to a pattern contained in a processed myoelectric signal).

In step **S701**, learning means of the determination means **222** learns at least two of a supervisory pattern of a motion of a jaw movement, a supervisory pattern of a motion of a tongue movement, and a supervisory pattern of a motion of a throat movement. The determination means **222** learns, for example, characteristics of at least two among a supervisory pattern of a motion of a jaw movement, a supervisory pattern of a motion of a tongue movement, and a supervisory pattern of a motion of a throat movement received via the interface unit **210,** and stores the characteristics in the database unit **250.** A supervisory pattern is a pattern contained in a processed myoelectric signal obtained by applying processing similar to the processing applied by the processing means **221** discussed above on a myoelectric signal obtained from a healthy individual performing a motion of a jaw movement, a motion of a tongue movement, or a motion of a throat movement. A characteristic of a supervisory pattern of a motion of a jaw movement, a supervisory pattern of a motion of a tongue movement, and a supervisory pattern of a motion of a throat movement can comprise, for example, a characteristic of a signal intensity distribution, a characteristic of a frequency distribution, and a characteristic of time series as explained in Table 1. It can be identified whether a muscle closest to the position where a measurement electrode is fitted is active from the characteristic of a signal intensity distribution. The difference in the primary active muscle can be identified from a characteristic of a frequency distribution. The temporal change in frequency and intensity of a myoelectric signal can be identified from a characteristic of time series. A pattern of a motion of a jaw movement, a pattern of a motion of a tongue movement, and a pattern of a motion of a throat movement can be clearly distinguished by combining these characteristics. A characteristic of a supervisory pattern of a motion of a jaw movement, a supervisory pattern of a motion of a tongue movement, and a supervisory pattern of a motion of a throat movement can also comprise, for example, a characteristic of a signal waveform. In this regard, learning means can also learn a supervisory pattern for no motion being performed in addition to the supervisory patterns discussed above.

In step **S702**, variable i is defined as i = 1.

In step **S703**, calculation means of the determination means **222** calculates the degree of similarity between a pattern contained in an output of the processing means **221** after processing in step **S502** (processed myoelectric signal) and the ith supervisory pattern. In this regard, the ith supervisory pattern is one of the supervisory patterns learned in step **S701**. For example, the first supervisory pattern can be a supervisory pattern of a mouth opening and closing motion of a jaw movement, the second supervisory pattern can be a supervisory pattern of a tapping motion of a jaw movement, the third supervisory pattern can be a supervisory pattern of a tongue raising motion of a tongue movement, the fourth supervisory pattern can be a supervisory pattern of a right side translation motion of a tongue movement, the fifth supervisory pattern can be a supervisory pattern of a left side translation motion of a tongue movement, the sixth supervisory pattern can be a supervisory pattern of a forward extension motion of a tongue movement, the seventh supervisory pattern can be a supervisory pattern of a left and right motion of a tongue movement, and the eight supervisory pattern can be a supervisory pattern of a dry swallowing motion of a throat movement. The calculation means can calculate the degree of similarity between an output of the processing means **221** (processed myoelectric signal) and ith supervisory pattern using a known similarity calculating technique such as normalized cross-correlation.

In step **S704**, it is judged whether the degree of similarity calculated in step **S703** exceeds a predetermined threshold value. The predetermined threshold value can be any value. The reliability of pattern determining processing (step **S503**) is higher for a higher predetermined threshold value. For example, when the degree of similarity calculated in step **S703** has a value of 0 to 1.0, the predetermined threshold value can be, for example, a value in the range of 0.4 to 0.9, such as 0.6. If it is judged in step **S704** that the degree of similarity calculated in step **S703** does not exceed a predetermined threshold value, the processing proceeds to step **S705**.

In step **S705**, the variable i is incremented. Steps **S703** to **S705** are repeated until it is judged that the degree of similarity exceeds the predetermined threshold value in step **S704**.

If it is judged that the degree of similarity calculated in step **S703** exceeds the predetermined threshold value in step **S704,** the processing proceeds to step **S706** and ends. This is because the specific pattern contained in an output of the processing means **221** can be determined to be a pattern of a motion indicated by the ith supervisory pattern whose degree of similarity has been judged to exceed the predetermined threshold value.

If none of the degrees of similarity calculated in step **S703** is determined to exceed the predetermined threshold value in step **S704,** a determination of "no motion" can be made to end the processing.

The example discussed above has explained an example of learning a supervisory pattern obtained from a healthy individual, but the present invention is not limited thereto. For example, a supervisory pattern to be learned can be a pattern contained in a processed myoelectric signal obtained from a myoelectric signal obtained from a patient with the same level or milder impairment than the subject, or a pattern contained in a processed myoelectric signal obtained from the subject himself/herself. This enables the determination means **222** to conduct evaluation that matches the movement level of the subject, leading to increased motivation of the subject. For example, comparing the subject's pattern with a pattern of a patient having a milder impairment than the subject and showing the result thereof clarifies the goal the subject should strive for, thus increasing the motivation of the subject.

In the example discussed above, the degree of similarity with each supervisory pattern was sequentially calculated until it was judged that the degree of similarity exceeded a predetermined threshold value, but the present invention is not limited thereto. For example, calculation of all of the degrees of similarity with each supervisory pattern together by using a neural network is also within the scope of the present invention.

. For example, the calculation means of the determination means **222** can be configured to calculate a degree of similarity between an output of the processing means **221** (processed myoelectric signal) and each supervisory pattern using a neural network in step **S702**' instead of step **S702**. In this regard, the neural network can be, for example, a multilayer perceptron having an input layer, a hidden layer, and an output layer. The output layer of the multilayer perceptron can comprise the same number of output nodes as the number of motions to be identified. The hidden layer of the multilayer perceptron can comprise any number of nodes. The weighting coefficient for each node of the hidden layer of the multilayer perceptron can be calculated based on a combination of each supervisory pattern and a teaching signal indicating what motion each supervisory pattern is in the learning processing in step **S701**. For example, the weighting coefficient for each node can be calculated so that a value of the output layer when a supervisory pattern is inputted into the input layer would be a value of an teaching signal corresponding to the supervisory pattern. This is performed, for example, by backpropagation. Each node of a neural network output layer whose weighting coefficient has been calculated in this manner becomes associated with the motion corresponding to each supervisory pattern.

For example, if the weighting coefficient for each node is calculated using a combination of a first supervisory pattern and an teaching signal (1.0, 0.0, 0.0, 0.0, 0.0, 0.0, 0.0, 0.0) indicating that the motion is a mouth opening and closing motion of a jaw movement, a combination of a second supervisory pattern and an teaching signal (0.0, 1.0, 0.0, 0.0, 0.0, 0.0, 0.0, 0.0) indicating that the motion is a tapping motion of a jaw movement, a combination of a third supervisory pattern and an teaching signal (0.0, 0.0, 1.0, 0.0, 0.0, 0.0, 0.0, 0.0) indicating that the motion is a tongue raising motion of a tongue movement, a combination of a fourth supervisory pattern and an teaching signal (0.0, 0.0, 0.0, 1.0, 0.0, 0.0, 0.0, 0.0) indicating that the motion is a right side translation motion of a tongue movement, a combination of a fifth supervisory pattern and an teaching signal (0.0, 0.0, 0.0, 0.0, 1.0, 0.0, 0.0, 0.0) indicating that the motion is a left side translation motion of a tongue movement, a combination of a sixth supervisory pattern and an teaching signal (0.0, 0.0, 0.0, 0.0, 0.0, 1.0, 0.0, 0.0) indicating that the motion is a forward extension motion of a tongue movement, a combination of a seventh supervisory pattern and an teaching signal (0.0, 0.0, 0.0, 0.0, 0.0, 0.0, 1.0, 0.0) indicating that the motion is a left and right movement motion of a tongue movement, and a combination of an eighth supervisory pattern and an teaching signal (0.0, 0.0, 0.0, 0.0, 0.0, 0.0, 0.0, 1.0) indicating that the motion is a dry swallowing motion of a throat movement when distinguishing 8 motions shown in Table 1 using a neural network having 8 output nodes, the first node of the output layer of the neural network is associated with a mouth opening and closing motion of a jaw movement, the second node is associated with a tapping motion of a jaw movement, the third node is associated with a tongue raising motion of a tongue movement, the fourth node is associated with a right side translation motion of a tongue movement, the fifth node is associated with a left side translation motion of a tongue movement, the sixth node is associated with a forward extension motion of a tongue movement, the seventh node is associated with a left and right movement motion of a tongue movement, and the eighth node is associated with a dry swallowing motion of a throat movement. Examples of ideal outputs of a neural network with weighting coefficient for each node calculated in this manner include an output of 1.0 by the first node of the output layer and an output of 0 by the other nodes when a processed myoelectric signal obtained from a myoelectric signal from a mouth opening and closing motion of a jaw movement is inputted. However, an ideal output is actually hardly ever obtained due to the effect of noise or the like that coexists with a myoelectric signal. In actuality, one or more nodes of the output layer would output a value in the range of 0 to 1. The value of each node of the output layer corresponds to the degree of similarity between a processed myoelectric signal that has been inputted and a supervisory pattern corresponding to a respective motion to which each node is associated.

For example, an output of (0.0, 0.2, 0.0, 0.8, 0.0, 0.0, 0.0, 0.0) indicates that a pattern contained in a processed myoelectric signal that has been inputted is slightly similar to a supervisory pattern corresponding to a tapping motion of a jaw movement associated with the second node, and more similar to a supervisory pattern corresponding to a right side translation motion of a tongue movement associated with the fourth node, and is not similar to supervisory patterns corresponding to motions associated with other nodes. In this regard, if the predetermined threshold value is for example 0.5, a pattern contained in a processed myoelectric signal that has been inputted is determined to be a pattern of a right side translation motion of a tongue movement.

For example, an output of (0.0, 0.0, 0.4, 0.5, 0.0, 0.7, 0.0, 0.4) indicates that the pattern contained in a processed myoelectric signal which has been inputted is slightly similar to supervisory patterns corresponding to a tongue raising motion of a tongue movement, a right side translation motion of a tongue movement, and a dry swallowing motion associated with the third, fourth, and eighth nodes, respectively, and is similar to a supervisory pattern corresponding to a forward extension motion of a tongue movement associated with the sixth node, and is not similar to supervisory patterns corresponding to movements associated with other nodes. In this regard, if the predetermined threshold value is for example 0.5, a pattern contained in a processed myoelectric signal that has been inputted is determined as a pattern of a right side translation motion of a tongue movement and a forward extension motion of a tongue movement.

In a preferred embodiment, processing in step **S503** is processing for determining which one or more of a pattern of a motion of a jaw movement, a pattern of a motion of a tongue movement, and a pattern of a motion of a throat movement correspond to a pattern contained in a processed myoelectric signal. A pattern of a motion of a jaw movement comprises a pattern of a mouth opening and closing motion, a pattern of a motion of a tongue movement comprises a pattern of a tongue raising motion, a pattern of a right side translation motion of the tongue, a pattern of a left side translation motion of the tongue, and a pattern of a forward extension motion of the tongue, and a pattern of a motion of a throat movement comprises a pattern of a dry swallowing motion. Identification of these patterns enables diagnosis of whether a subject has a swallowing impairment or not or diagnosis of a motion level in a swallowing motion. In a more preferred embodiment, processing in step **S503** also identifies a pattern of a tapping motion in a jaw movement, and a pattern of a left and right movement motion of the tongue in a tongue movement in addition to the patterns of motions discussed above. This can improve the precision of the diagnosis of whether a subject has a swallowing impairment or not or diagnosis of a motion level in a swallowing motion.

In step **S701** in a preferred embodiment, the learning means of the determination means **222** learns a supervisory pattern of a motion of a jaw movement including a supervisory pattern of a mouth opening and closing motion, supervisory patterns of motions of a tongue movement including a supervisory pattern of a tongue raising motion, a supervisory pattern of a right side translation motion of the tongue, a supervisory pattern of a left side translation motion of the tongue, and a supervisory pattern of a forward extension motion of the tongue, and a supervisory pattern of a motion of a throat movement including a supervisory pattern of a dry swallowing motion. The determination means **222** learns, for example, characteristics of a supervisory pattern of a motion of a jaw movement, a supervisory pattern of a motion of a tongue movement, and a supervisory pattern of a motion of a throat movement received via the interface unit **210,** and stores the characteristics in the database unit **250.** The characteristics of a supervisory pattern of a motion of a jaw movement, a supervisory pattern of a motion of a tongue movement, and a supervisory pattern of a motion of a throat movement can comprise, for example, a characteristic of a signal intensity distribution, a characteristic of a frequency distribution, and a characteristic of time series, as explained in Table 1. It can be identified whether a muscle closest to a position where measurement electrodes are fitted is active with a characteristic of a signal intensity distribution. A difference in the primary active muscle can be identified with a characteristic of a frequency distribution. A temporal change in the frequency and intensity of a myoelectric signal can be identified with a characteristic of time series. A pattern of a motion of a jaw movement, a pattern of a motion of a tongue movement, and a pattern of a motion of a throat movement can be clearly distinguished with a combination of these characteristics. A characteristic of a supervisory pattern of a motion of a jaw movement, a supervisory pattern of a motion of a tongue movement, and a supervisory pattern of a motion of a throat movement can also comprise, for example, a characteristic of a signal waveform. In this regard, learning means can learn a supervisory pattern for no motion being performed in addition to the aforementioned supervisory patterns.

In step **S702** in a preferred embodiment, variable i is defined as i = 1.

In step **S703** in a preferred embodiment, calculation means of the determination means **222** calculates the degree of similarity between a pattern contained in an output of the processing means **221** after applying processing in step **S502** (processed myoelectric signal) and the ith supervisory pattern. In this regard, the ith supervisory pattern is one of the supervisory patterns learned in step **S701**. For example, the first supervisory pattern is a supervisory pattern of a mouth opening and closing motion of a jaw movement, the second supervisory pattern is a supervisory pattern of a tongue raising motion of a tongue movement, the third supervisory pattern is a supervisory pattern of a right side translation motion of a tongue movement, the fourth supervisory pattern is a supervisory pattern of a left side translation motion of a tongue movement, the fifth supervisory pattern is a supervisory pattern of a forward extension motion of a tongue movement, and the sixth supervisory pattern is a supervisory pattern of a dry swallowing motion of a throat movement. The calculation means can calculate the degree of similarity between an output of the processing means **221** (processed myoelectric signal) and the ith supervisory pattern by using a known similarity calculating technique such as normalized cross-correlation.

In step **S704** in a preferred embodiment, it is judged whether the degree of similarity calculated in step **S703** exceeds a predetermined threshold value. The predetermined threshold value can be any value. The reliability of pattern determining processing (step **S503**) is higher for a higher predetermined threshold value. For example, when the degree of similarity calculated in step **S703** has a value of 0 to 1.0, the predetermined threshold value can be, for example, a value in the range of 0.4 to 0.9, such as 0.6. If it is judged in step **S704** that the degree of similarity calculated in step **S703** does not exceed a predetermined threshold value, the processing proceeds to step **S705**.

In step **S705** in a preferred embodiment, the variable i is incremented. Steps **S703** to **S705** are repeated until it is judged that the degree of similarity exceeds the predetermined threshold value in step **S704**.

If it is judged that the degree of similarity calculated in step **S703** exceeds the predetermined threshold value in step **S704** in a preferred embodiment, the processing proceeds to step **S706** and ends. This is because the specific pattern contained in an output of the processing means **221** can be determined to be a pattern of a motion indicated by the ith supervisory pattern whose degree of similarity has been judged to exceed the predetermined threshold value.

In the preferred embodiment discussed above, the calculation means of the determination means **222** can be configured to calculate a degree of similarity between an output of the processing means **221** (processed myoelectric signal) and each supervisory pattern using a neural network in step **S702**' instead of step **S702**.

The above examples have explained that the learning means of the determination means **222** learns in step **S701** of step **S503**, but the learning means of the determination means **222** may learn in advance at least two of a supervisory pattern of a motion of a jaw movement, a supervisory pattern of a motion of a tongue movement, and a supervisory pattern of a motion of a throat movement by the processing similar to that in step **S701**, before starting processing **500.** In such a case, step **S701** is omitted in the processing in step **S503**. Even when the calculation means of the determination means **222** calculates a degree of similarity between an output of the processing means **221** (processed myoelectric signal) and each supervisory pattern using a neural network in step **S702**' instead of step **S702**, the learning means of the determination means **222** can similarly learn a supervisory pattern of a motion to be identified using a neural network before starting processing **500.**

The learning processing comprises, for example, detecting, by a myoelectric device, a myoelectric signal indicating the activity of a muscle when a subject without an impairment in a predetermined movement comprising at least two of a jaw movement, a tongue movement, and a throat movement (i.e., healthy individual) performs a predetermined movement, receiving a detected myoelectric signal by the processor unit **220,** outputting, by the processor unit **220,** a processed myoelectric signal by applying predetermined processing on the received myoelectric signal, and learning, by the processor unit **220,** a pattern contained in the processed myoelectric signal as a pattern in a predetermined movement. The predetermined processing is, for example, the same processing as the processing by the processing means **221** discussed above.

Learning processing can be performed by using, for example, a supervisory pattern of a motion of a jaw movement, a supervisory pattern of a motion of a tongue movement, and a supervisory pattern of a motion of a throat movement, preferably a supervisory pattern of a mouth opening and closing motion, a supervisory pattern of a tongue raising motion, a supervisory pattern of a right side translation motion of the tongue, a supervisory pattern of a left side translation motion of the tongue, a supervisory pattern of a forward extension motion of the tongue, and a supervisory pattern of a dry swallowing motion. The determination means **222** subjected to learning processing using these patterns can be used as a classifier, which can identify a pattern of a mouth opening closing motion, a pattern of a tongue raising motion, a pattern of a right side translation motion of the tongue, a pattern of a left side translation motion of the tongue, a pattern of a forward extension motion of the tongue, and a pattern of a dry swallowing motion that are useful as an indicator in diagnosis of a swallowing impairment. More preferably, this can be performed using a supervisory pattern of a mouth opening and closing motion, a supervisory pattern of a tapping motion, a supervisory pattern of a tongue raising motion, a supervisory pattern of a right side translation motion of the tongue, a supervisory pattern of a left side translation motion of the tongue, a supervisory pattern of a forward extension motion of the tongue, a supervisory pattern of a left and right translation motion of the tongue, and a supervisory pattern of a dry swallowing motion. The determination means **222** subjected to learning processing using these patterns can be used as a classifier, which can identify a pattern of a mouth opening closing motion, a pattern of a tapping motion, a pattern of a tongue raising motion, a pattern of a right side translation motion of the tongue, a pattern of a left side translation motion of the tongue, a pattern of a forward extension motion of the tongue, a pattern of a left and right translation motion of the tongue, and a pattern of a dry swallowing motion that are further useful as an indicator in diagnosis of a swallowing impairment.

Figure **7B** depicts an example of processing in step **S513** (processing performed by determination means **222** for determining a motion level in a jaw movement, tongue movement, or throat movement of a subject based on a pattern contained in a processed myoelectric signal).

In step **S711**, the learning means of the determination means **222** learns a supervisory pattern of a motion corresponding to a predetermined motion among a supervisory pattern of a motion of a jaw movement, a supervisory pattern of a motion of a tongue movement, and a supervisory pattern of a motion of a throat movement. For example, the determination means **222** learns a supervisory pattern of a motion of a jaw movement, a supervisory pattern of a motion of a tongue movement, or a supervisory pattern of a motion of a throat movement received through the interface unit **210,** and stores the pattern in the database unit **250.** The supervisory pattern is a pattern contained in a processed myoelectric signal obtained by applying the same processing as the processing by the processing means **221** discussed above on a myoelectric signal obtained from a healthy individual performing a motion of a jaw movement, a motion of a tongue movement, or a motion of throat movement. The characteristics of a supervisory pattern of a motion of a jaw movement, a supervisory pattern of a motion of a tongue movement, and a supervisory pattern of a motion of a throat movement can comprise, for example, a characteristic of a signal intensity distribution, a characteristic of a frequency distribution, and a characteristic of time series, as explained in Table 1. It can be identified whether a muscle closest to a position where measurement electrodes are fitted is active with a characteristic of a signal intensity distribution. A difference in the primary active muscle can be identified with a characteristic of a frequency distribution. A temporal change in the frequency and intensity of a myoelectric signal can be identified with a characteristic of time series. A pattern of a motion of a jaw movement, a pattern of a motion of a tongue movement, and a pattern of a motion of a throat movement can be clearly distinguished with a combination of these characteristics. A characteristic of a supervisory pattern of a motion of a jaw movement, a supervisory pattern of a motion of a tongue movement, and a supervisory pattern of a motion of a throat movement can also comprise, for example, a characteristic of a signal waveform.

In step **S712**, the calculation means of the determination means **222** can calculate a degree of similarity between a pattern contained in an output of the processing means **221** after applying the processing in step **S512** (processed myoelectric signal) and a supervisory pattern. The calculation means can calculate a degree of similarity between an output of the processing means **221** (processed myoelectric signal) and a supervisory pattern by using a known similarity calculating technique such as normalized cross-correlation. Alternatively, the calculation means can be configured to calculate a degree of similarity between an output of the processing means **221** (processed myoelectric signal) and a supervisory pattern using the neural network discussed above.

The degree of similarity discussed above can be utilized, for example, for determining a motion level of a subject by the determination means **222**. For example, when the degree of similarity is represented as 0 to 1.0, a degree of similarity of 1.0 indicates that a motion level is the same level as that of a healthy individual. A degree of similarity at or below a predetermined threshold value indicates that a subject is unable to perform the motion. A degree of similarity greater than a predetermined threshold value but less than 1.0 indicates that a subject is able to perform the motion, but not as high as that of a healthy individual. If for example the predetermined threshold value is 0.5, a degree of similarity of 0.6 indicates that the motion level is about 20% compared to a motion level of a healthy individual, a degree of similarity of 0.7 indicates that the motion level is about 40% compared to a motion level of a healthy individual, a degree of similarity of 0.8 indicates that the motion level is about 60% compared to a motion level of a healthy individual, and a degree of similarity of 0.9 indicates that the motion level is about 80% compared to a motion level of a healthy individual.

If, for example, a subject is asked to perform a dry swallowing motion of a throat movement to determine a degree of similarity between a pattern of a motion contained in a myoelectric signal obtained therefrom and a supervisory pattern of dry swallowing, a degree of similarity of 1.0 indicates that a dry swallowing motion level is the same level as that of a healthy individual. A degree of similarity at or below a predetermined threshold value indicates that a subject is unable to perform a dry swallowing motion. A degree of similarity that is greater than a predetermined threshold value but less than 1.0 indicates that a subject is able to perform a dry swallowing motion, but has an impairment in a throat movement and a dry swallowing motion level is not as high as that of a healthy individual. If for example the predetermined threshold value is 0.5, a degree of similarity of 0.6 indicates that the motion level is about 20% compared to a dry swallowing motion level of a healthy individual, a degree of similarity of 0.7 indicates that the motion level is about 40% compared to a dry swallowing motion level of a healthy individual, a degree of similarity of 0.8 indicates that the motion level is about 60% compared to a dry swallowing motion level of a healthy individual, and a degree of similarity of 0.9 indicates that the motion level is about 80% compared to a dry swallowing motion level of a healthy individual.

A motion level based on a degree of similarity can be presented to a subject, for example, by displaying the level on a monitor as a numerical value or by expressing the movement with an avatar of the subject. This enables subjects to see their own impairment level relative to a healthy individual. Alternatively, a subject in rehabilitation can check the level of improvement in rehabilitation by having motion levels presented over time.

The degree of similarity discussed above can also be utilized, for example, for evaluating the presence or absence of an impairment in a movement of a subject. For example, if it is judged that a degree of similarity calculated in step **S712** does not exceed a predetermined threshold value upon judgment of whether the degree of similarity calculated in step **S712** exceeds the predetermined threshold value, the processing proceeds to step **S714** and a predetermined movement of a subject is evaluated as having an impairment. If it is judged that a degree of similarity calculated in step **S712** exceeds a predetermined threshold value, the processing proceeds to step **S715** and a predetermined movement of a subject is evaluated as having no impairment. The predetermined threshold value can be any value. The reliability of processing for evaluating the presence of absence of an impairment (step **S513**) is higher for a higher predetermined threshold value. For example, when the degree of similarity calculated in step **S712** has a value of 0 to 1.0, the predetermined threshold value can be, for example, a value in the range of 0.4 to 0.9, such as 0.7.

If for example a subject is asked to perform a dry swallowing motion of a throat movement to determine a degree of similarity between a pattern of a movement contained in a myoelectric signal obtained therefrom and a supervisory pattern of dry swallowing, a degree of similarity that is no more than 0.6, when a predetermined threshold value is 0.6, indicates that there is an impairment in a dry swallowing motion, and a degree of similarity that is greater than 0.6 indicates that there is no impairment in a dry swallowing motion.

The presence or absence of an impairment in a movement of a subject can be evaluated, for example, by asking the subject to perform a plurality of motions. For example, processing can be configured so that if, among the plurality of motions, the number of motions with a degree of similarity greater than a predetermined threshold value is less than a predetermined number, this indicates that there is an impairment in the movement in the subject, and if the number of motions with a degree of similarity greater than a predetermined threshold value is greater than a predetermined number, this indicates that there is no impairment in the movement in the subject.

If for example a subject is asked to perform a mouth opening and closing motion of the jaw, a tongue raising motion, a right side translation motion of the tongue, a left side translation motion of the tongue, a forward extension motion of the tongue, and a dry swallowing motion to determine the degrees of similarity between a pattern of motion contained in a myoelectric signal for each motion and each of a supervisory pattern of a mouth opening and closing motion of the jaw, a supervisory pattern of a tongue raising motion, a supervisory pattern of a right side translation motion of the tongue, a supervisory pattern of a left side translation motion of the tongue, a supervisory pattern of a forward extension motion of the tongue, and a supervisory pattern of a dry swallowing motion, the number of degrees of similarity that are greater than the predetermined threshold value among the degrees of similarity of the six motions of less than 4 indicates, when the predetermined number is 4, that there is an impairment in a swallowing movement or a chewing movement of the subject. A number of degrees of similarity that are greater than the predetermined threshold value among the degree of similarities of the six motions of 4 or greater indicates, when the predetermined number is 4, that there is no impairment in a swallowing movement or a chewing movement of the subject.

The above examples have explained that the learning means of the determination means **222** learns in step **S711** of step **S513**, but the learning means of the determination means **222** may learn in advance a supervisory pattern of a motion corresponding to a predetermined motion among a supervisory pattern of a motion of a jaw movement, a supervisory pattern of a motion of a tongue movement, and a supervisory pattern of a motion of a throat movement by the same processing as step S711, before starting processing **500**. In such a case, step **S711** is omitted in the processing in step **S513**. Even when the calculation means of the determination means **222** calculates a degree of similarity between an output of the processing means **221** (processed myoelectric signal) and a supervisory pattern using a neural network in step **S712**, the learning means of the determination means **222** can similarly learn a supervisory pattern of a motion to be identified using a neural network before starting processing **500**.

The learning processing comprises, for example, detecting, by a myoelectric device, a myoelectric signal indicating the activity of a muscle when a subject without an impairment in a predetermined movement comprising a jaw movement, a tongue movement, or a throat movement (i.e., healthy individual) performs a predetermined movement, receiving a detected myoelectric signal by the processor unit **220**, outputting, by the processor unit **220**, a processed myoelectric signal by applying predetermined processing on the received myoelectric signal, and learning, by the processor unit **220**, a pattern contained in a processed myoelectric signal as a pattern in a predetermined movement. The predetermined processing is, for example, the same processing as the processing by the processing means **221** discussed above.

Learning processing can be performed by using, for example, a supervisory pattern of a motion of a jaw movement, a supervisory pattern of a motion of a tongue movement, and a supervisory pattern of a motion of a throat movement, preferably a supervisory pattern of a mouth opening and closing motion, a supervisory pattern of a tongue raising motion, a supervisory pattern of a right side translation motion of the tongue, a supervisory pattern of a left side translation motion of the tongue, a supervisory pattern of a forward extension motion of the tongue, and a supervisory pattern of a dry swallowing motion. The determination means **222** subjected to learning processing using these patterns can be used as a classifier, which can identify a pattern of a mouth opening closing motion, a pattern of a tongue raising motion, a pattern of a right side translation motion of the tongue, a pattern of a left side translation motion of the tongue, a pattern of a forward extension motion of the tongue, and a pattern of a dry swallowing motion that are useful as an indicator in diagnosis of a swallowing impairment. More preferably, this can be performed using a supervisory pattern of a mouth opening and closing motion, a supervisory pattern of a tapping motion, a supervisory pattern of a tongue raising motion, a supervisory pattern of a right side translation motion of the tongue, a supervisory pattern of a left side translation motion of the tongue, a supervisory pattern of a forward extension motion of the tongue, a supervisory pattern of a left and right translation motion of the tongue, and a supervisory pattern of a dry swallowing motion. The determination means **222** subjected to learning processing using these patterns can be used as a classifier, which can identify a pattern of a mouth opening closing motion, a pattern of a tapping motion, a pattern of a tongue raising motion, a pattern of a right side translation motion of the tongue, a pattern of a left side translation motion of the tongue, a pattern of a forward extension motion of the tongue, a pattern of a left and right translation motion of the tongue, and a pattern of a dry swallowing motion that are further useful as an indicator in diagnosis of a swallowing disorder.

While one supervisory pattern with a degree of similarity exceeding a predetermined threshold value was determined in an example depicted in Figure **7A**, determination of a plurality of supervisory patterns with a degree of similarity exceeding a predetermined threshold value is also within the scope of the present invention. For example, the processing can be configured so that step **S703** to step **S704** are performed on all learned supervisory patterns to determine one or more supervisory patterns judged to have a degree of similarity exceeding a predetermined threshold value, and determine each of the plurality of patterns contained in a processed myoelectric signal is a pattern of motion indicated by each of the determined one or more supervisory patterns. For example in step **S702'**, the processing can be configured to determine one or more supervisory patterns judged to have a degree of similarity exceeding a predetermined threshold value from an output of a neural network and determine that each of the plurality of patterns contained in a processed myoelectric signal is a pattern of a motion indicated by each of one or more of the determined supervisory patterns. For example, the processing can be configured so that step **S703** to step **S704** are performed on all learned supervisory patterns to determine one optimal supervisory pattern from one or more supervisory patterns judged to have a degree of similarity exceeding a predetermined threshold value. For example in step **S702'**, the processing can be configured to determine an optimal supervisory pattern from one or more supervisory patterns judged to have a degree of similarity exceeding a predetermined threshold value from an output of a neural network. An optimal supervisory pattern can be determined, for example, by determining a supervisory pattern with the highest degree of similarity. An optimal supervisory pattern can be determined, for example, by comparison with past data. For example, the tendency of a motion is estimated from results recognized in the most recent recognition processing to determine a supervisory pattern of a motion in alignment with the tendency as an optimal supervisory pattern. For example, when a degree of similarity is judged to exceed a predetermined threshold value for a supervisory pattern of a tongue raising motion and a supervisory pattern of a forward extension motion after a pattern of a tongue raising motion has been recognized in the three most recent recognition processing, a supervisory pattern of a tongue raising motion is determined as an optimal supervisory pattern from the result of the most recent recognition processing. While an optimal supervisory pattern can be determined by any approach, the tendency of a motion is preferably estimated from a result recognized in the most recent recognition processing to determine a supervisory pattern of a motion in alignment with the tendency as an optimal supervisory pattern, because an error in determining patterns can be compensated by reference to past data.

The present invention is not limited to the aforementioned embodiments. It is understood that the scope of the present invention should be interpreted solely by the scope of the claims. It is understood that those skilled in the art can implement an equivalent scope, based on the descriptions of the invention and common general knowledge, from the descriptions of the specific preferred embodiments of the invention.

### [Industrial Applicability]

The present invention has utility as an invention providing a system, method, and program for recognizing myoelectric signal-originating motions comprising at least two of a motion of a jaw movement, a motion of a tongue movement, and a motion of a throat movement.

### [Reference Signs List]

- **10**: System
- **100**: Myoelectric device
- **111**, **112**: Myoelectric sensor
- **113**, **113**': Pair of measurement electrodes
- **114**, **114'**: Pair of measurement electrodes
- **115**: Transmission unit **115**
- **200**: Computer apparatus
- **210**: Interface unit
- **220**: Processor unit
- **230**: Memory unit
- **250**: Database unit

## Claims

1. A system for evaluating a movement of a subject, the system comprising:
detection means for detecting a myoelectric signal indicating an activity of a muscle when a subject performs a movement intended to be a predetermined movement comprising a jaw movement, a tongue movement, or a throat movement;
processing means for outputting a processed myoelectric signal by applying predetermined processing on the detected myoelectric signal; and
determination means for determining a motion level in the predetermined movement of the subject, based on a pattern contained in the processed myoelectric signal.

2. The system of claim 1, wherein the predetermined processing comprises frequency analysis processing, and the processed myoelectric signal comprises time series data for a frequency distribution of the myoelectric signal.

3. The system of claim 2, wherein the frequency analysis processing comprises a short-time FFT.

4. The system of claim 2 or 3, wherein the predetermined processing further comprises signal intensity extraction processing, and the processed myoelectric signal further comprises time series data for signal intensities of the myoelectric signal.

5. The system of any one of claims 1 to 4, wherein the determination means is subjected to learning processing using a supervisory pattern of a motion corresponding to the predetermined movement among a supervisory pattern of a motion of a jaw movement, a supervisory pattern of a motion of a tongue movement, and a supervisory pattern of a motion of a tongue movement, so that a degree of similarity to a myoelectric signal generated when there is no impairment in the predetermined movement is outputted,
wherein the determination means comprises:
calculation means for calculating a degree of similarity between the pattern contained in the processed myoelectric signal and the supervisory pattern.

6. The system of claim 5, wherein the determination means further evaluates the presence or absence of an impairment in the predetermined movement based on the degree of similarity.

7. The system of claim 5 or 6, wherein the learning processing is applied using a supervisory pattern of a motion of a jaw movement, a supervisory pattern of a motion of a tongue movement, and a supervisory pattern of a motion of a throat movement.

8. The system of claim 7, wherein the learning processing is applied using at least a supervisory pattern of a mouth opening and closing motion associated with the motion of a jaw movement, at least a supervisory pattern of a tongue raising motion, a supervisory pattern of a right side translation motion of the tongue, a supervisory pattern of a left side translation motion of the tongue, and a supervisory pattern of a forward extension motion of the tongue associated with the motion of a tongue movement, and at least a supervisory pattern of a dry swallowing motion associated with the motion of a throat movement.

9. The system of claim 8, wherein the learning processing is applied by further using a supervisory pattern of a tapping motion associated with the motion of a jaw movement and a supervisory pattern of a left and right translation motion of the tongue associated with the motion of a tongue movement.

10. The system of any one of claims 1 to 9,
wherein the detection means comprises a myoelectric sensor comprising two pairs of measurement electrodes, and
wherein the two pairs of measurement electrodes are configured to be able to detect a myoelectric signal indicating an activity of a jaw muscle, a tongue muscle, or a throat muscle.

11. A method for evaluating a movement of a subject, the method comprising:
detecting a myoelectric signal indicating an activity of a muscle when a subject performs a movement intended to be a predetermined movement comprising a jaw movement, a tongue movement, or a throat movement;
outputting a processed myoelectric signal by applying predetermined processing on the detected myoelectric signal; and
determining a motion level in the predetermined movement of the subject, based on a pattern contained in the processed myoelectric signal.

12. A program for evaluating a movement of a subject, the program executed in a computer apparatus comprising a processor unit, wherein the program, when executed, causes the processor unit to perform processing comprising:
receiving a myoelectric signal indicating an activity of a muscle when a subject performs a movement intended to be a predetermined movement comprising a jaw movement, a tongue movement, or a throat movement;
outputting a processed myoelectric signal by applying predetermined processing on the received myoelectric signal; and
determining a motion level in the predetermined movement of the subject, based on a pattern contained in the processed myoelectric signal.

13. A classifier used for evaluating a movement of a subject, the classifier comprising:
detection means for detecting a myoelectric signal indicating an activity of a muscle when a subject with no impairment in a predetermined movement comprising a jaw movement, a tongue movement, or a throat movement performs the predetermined movement;
processing means for outputting a processed myoelectric signal by applying predetermined processing on the detected myoelectric signal; and
learning means for learning a pattern contained in the processed myoelectric signal as a pattern in the predetermined movement.

14. The classifier of claim 13, wherein the predetermined movement comprises a jaw movement, a tongue movement, and a throat movement.

15. The classifier of claim 14, wherein the predetermined movement comprises at least a mouth opening and closing motion associated with a motion of the jaw movement, at least a tongue raising motion, a right side translation motion of the tongue, a left side translation motion of the tongue, and a forward extension motion of the tongue associated with a motion of the tongue movement, and at least a dry swallowing motion associated with a motion of the throat movement.

16. The classifier of claim 15, wherein the predetermined movement further comprises a tapping motion associated with a motion of the jaw movement and a left and right translation motion of the tongue associated with a motion of the tongue movement.

17. A method for constructing a classifier used for evaluating a movement of a subject, the method comprising:
detecting a myoelectric signal indicating an activity of a muscle when a subject with no impairment in a predetermined movement comprising a jaw movement, a tongue movement, or a throat movement performs the predetermined movement;
outputting a processed myoelectric signal by applying predetermined processing on the detected myoelectric signal; and
learning a pattern contained in the processed myoelectric signal as a pattern in the predetermined movement.

18. A program for constructing a classifier used for evaluating a movement of a subject, the program executed in a computer apparatus comprising a processor unit, wherein the program, when executed, causes the processor unit to perform processing comprising:
receiving a myoelectric signal indicating an activity of a muscle when a subject with no impairment in a predetermined movement comprising a jaw movement, a tongue movement, or a throat movement performs the predetermined movement;
outputting a processed myoelectric signal by applying predetermined processing on the detected myoelectric signal; and
learning a pattern contained in the processed myoelectric signal as a pattern in the predetermined movement.
